(19) Europäisches Patentamt
European Patent Office
Office européen des brevets

(11) **EP 4 691 345 A1**

(12) **EUROPEAN PATENT APPLICATION**
published in accordance with Art. 153(4) EPC

(43) Date of publication:
**11.02.2026 Bulletin 2026/07**

(21) Application number: **24778748.4**

(22) Date of filing: **13.02.2024**

(51) International Patent Classification (IPC):
**A61B 3/14** $^{(2006.01)}$     **A61B 3/12** $^{(2006.01)}$

(52) Cooperative Patent Classification (CPC):
**A61B 3/12; A61B 3/14**

(86) International application number:
**PCT/JP2024/004745**

(87) International publication number:
**WO 2024/202603 (03.10.2024 Gazette 2024/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **30.03.2023 JP 2023055524**

(71) Applicant: Topcon Corporation
Tokyo 174-8580 (JP)

(72) Inventors:
• SAKAI Jun
Tokyo 174-8580 (JP)
• MORISHIMA Syunichi
Tokyo 174-8580 (JP)

(74) Representative: **Gagel, Roland
Patentanwalt Dr. Roland Gagel
Landsberger Strasse 480a
81241 München (DE)**

(54) **OCULAR FUNDUS IMAGING DEVICE**

(57) A fundus imaging apparatus includes two or more curved mirrors, an illumination optical system, and an image sensor. The illumination optical system includes a slit with an opening configured to be disposed at a fundus conjugate position. Here, the fundus conjugate position is substantially conjugate optically to a fundus of an eye to be examined. The illumination optical system is configured to irradiate slit-shaped illumination light, that is generated by irradiating light from a light source onto the slit, onto the fundus through the two or more curved mirrors. The image sensor is configured to be disposed at the fundus conjugate position and to receive returning light from the eye to be examined. At least one of both ends of the opening in a longitudinal direction is displaced in a shorter direction of the opening with reference to the longitudinal direction passing through a center of the opening.

FIG. 1

**Description**

[TECHNICAL FIELD]

The present invention relates to a fundus imaging apparatus.

[BACKGROUND ART]

Fundus imaging apparatuses (fundus photography apparatuses) used for screening or treatment of eye diseases are expected to be capable of easily imaging (observing) the fundus of the eye to be examined with a wide field of view. Specifically, the fundus imaging apparatuses capable of imaging the fundus of the eye to be examined at a wide imaging angle of view of more than 80 degrees in a single imaging are expected. As such fundus imaging apparatuses, scanning laser ophthalmoscopes (SLOs) are known. SLO is an apparatus configured to form an image of the fundus by scanning the fundus with light to detect returning light of the light with a light receiving device.

For example, Patent Document 1 discloses a scanning ophthalmoscope that can scan a retina at a wide angle by moving a two-dimensional collimated scan using a polygon mirror and a plane mirror on the eye to be examined using a scan transfer means.

For example, Patent Document 2 and Patent Document 3 disclose a fundus imaging apparatus that can acquire high-contrast images with a simple configuration, by combining a fundus scanning using slit-shaped illumination light and a rolling shutter. In particular, Patent Document 3 discloses a method of acquiring wide-angle fundus images of the eye to be examined by scanning the fundus of the eye to be examined with the slit-shaped illumination light through two ellipsoidal concave mirrors.

Further, Patent Document 4 discloses an ophthalmic apparatus provided with a projection diaphragm having an opening that has a portion with a width wider than the width of the opening at the optical axis. Here, the ophthalmic apparatus performs fundus scanning using illumination light that has passed through the projection diaphragm.

[PRIOR ART DOCUMENTS]

[PATENT DOCUMENTS]

[PATENT DOCUMENT 1] Japanese Unexamined Patent Application Publication (Translation of PCT Application) No. 2009-543585
[PATENT DOCUMENT 2] US Patent No. 7,831,106
[PATENT DOCUMENT 3] WO 2022/124170
[PATENT DOCUMENT 4] Japanese Unexamined Patent Application Publication (Translation of PCT Application) No. 2017-526474

[SUMMARY OF THE INVENTION]

[PROBLEMS TO BE SOLVED BY THE INVENTION]

The method disclosed in Patent Document 1 requires a high speed scanner such as a polygon mirror. However, high speed scanners capable of scanning at wide angles are very expensive.

Further, in case of imaging the fundus at a wide angle, an optical path of illumination light and an optical path of returning light of the illumination light are desirable to be spatially divided at a branch point at a position conjugate to a pupil of the eye to be examined. The method disclosed in Patent Document 2 has difficulty in pupil dividing. In addition, it is extremely difficult to limit the light flux for illuminating a wide range on the fundus and the light flux of the returning light within an effective diameter of the optical system while maintaining the working distance.

Furthermore, the method disclosed in Patent Document 3 makes it possible to image the fundus of the eye to be examined at a wide angle at low cost and with a simple configuration. However, due to the susceptibility to aberrations of the ellipsoidal mirror, advanced optical design becomes necessary.

Furthermore, in the method disclosed in Patent Document 4, not only returning light from the fundus but also unnecessary light is received. As a result, contrast reduction, flare, and/or ghost caused by the reception of the unnecessary light occur, and the image quality of the image of the fundus deteriorates.

These problems are common not only when acquiring wide-angle images of the fundus, but also when acquiring images of the fundus.

The present invention has been made in view of such circumstances, and one of objects of the present invention is to provide a new technique for acquiring higher-quality images of a fundus of an eye to be examined at low cost and with a simple configuration, without requiring advanced optical design.

[MEANS OF SOLVING THE PROBLEMS]

One aspect of embodiments is a fundus imaging apparatus, including: two or more curved mirrors; an illumination optical system including a slit with an opening configured to be disposed at a fundus conjugate position, the fundus conjugate position being substantially conjugate optically to a fundus of an eye to be examined, and configured to irradiate slit-shaped illumination light, that is generated by irradiating light from a light source onto the slit, onto the fundus through the two or more curved mirrors; and an image sensor configured to be disposed at the fundus conjugate position and to receive returning light from the eye to be examined, wherein at least one of both ends of the opening in a longitudinal direction is displaced in a shorter direction of the opening with reference to the longitudinal direction passing through a center of the opening.

[EFFECTS OF THE INVENTION]

According to the present invention, a new technique for acquiring higher-quality images of a fundus of an eye to be examined at low cost and with a simple configuration, without requiring advanced optical design can be provided.

[BRIEF EXPLANATION OF THE DRAWINGS]

[FIG. 1] FIG. 1 is a schematic diagram illustrating an example of a configuration of an optical system of a fundus imaging apparatus according to embodiments.
[FIG. 2] FIG. 2 is a schematic diagram illustrating an example of a configuration of an optical system of the fundus imaging apparatus according to the embodiments.
[FIG. 3] FIG. 3 is a schematic diagram illustrating an example of a configuration of an optical system of the fundus imaging apparatus according to the embodiments.
[FIG. 4] FIG. 4 is a schematic diagram for explaining a configuration of the optical system of the fundus imaging apparatus of the embodiments.
[FIG. 5] FIG. 5 is a schematic diagram illustrating an example of a configuration of an optical system of the fundus imaging apparatus according to the embodiments.
[FIG. 6] FIG. 6 is a schematic diagram illustrating an example of a configuration of an optical system of the fundus imaging apparatus according to the embodiments.
[FIG. 7] FIG. 7 is a schematic diagram illustrating an example of a configuration of an optical system of the fundus imaging apparatus according to the embodiments.
[FIG. 8] FIG. 8 is a schematic diagram illustrating an example of a configuration of the optical system of the fundus imaging apparatus according to the embodiments.
[FIG. 9A] FIG. 9A is a schematic diagram for explaining a configuration of an optical system of a fundus imaging apparatus according to a comparative example of the embodiments.
[FIG. 9B] FIG. 9B is a schematic diagram for explaining a configuration of the optical system of the fundus imaging apparatus according to the comparative example of the embodiments.
[FIG. 9C] FIG. 9C is a schematic diagram for explaining a configuration of the optical system of the fundus imaging apparatus according to the comparative example of the embodiments.
[FIG. 10A] FIG. 10A is a schematic diagram illustrating an example of a configuration of an optical system of the fundus imaging apparatus according to the embodiments.
[FIG. 10B] FIG. 10B is a schematic diagram illustrating an example of a configuration of the optical system of the fundus imaging apparatus according to the comparative example of the embodiments.
[FIG. 10C] FIG. 10C is a schematic diagram for explaining a configuration of the optical system of the fundus imaging apparatus according to the comparative example of the embodiments.
[FIG. 10D] FIG. 10D is a schematic diagram for explaining a configuration of the optical system of the fundus imaging apparatus according to the comparative example of the embodiments.
[FIG. 10E] FIG. 10E is a schematic diagram for explaining a configuration of the optical system of the fundus imaging apparatus according to the comparative example of the embodiments.
[FIG. 11A] FIG. 11A is a schematic diagram for explaining a configuration of the optical system of the fundus imaging apparatus of the embodiments.

[FIG. 11B] FIG. 11B is a schematic diagram for explaining a configuration of the optical system of the fundus imaging apparatus of the embodiments.

[FIG. 12A] FIG. 12A is a schematic diagram for explaining an operation of the fundus imaging apparatus according to the embodiments.

[FIG. 12B] FIG. 12B is a schematic diagram for explaining an operation of the fundus imaging apparatus according to the embodiments.

[FIG. 13] FIG. 13 is a schematic diagram illustrating an example of a configuration of a processing system of the fundus imaging apparatus according to the embodiments.

[FIG. 14] FIG. 14 is a flowchart illustrating an example of the operation of the fundus imaging apparatus according to the embodiments.

[FIG. 15] FIG. 15 is a schematic diagram illustrating an example of a configuration of an optical system of the fundus imaging apparatus according to a first modification example of the embodiments.

[FIG. 16] FIG. 16 is a schematic diagram illustrating an example of a configuration of an optical system of the fundus imaging apparatus according to the first modification example of the embodiments.

[FIG. 17] FIG. 17 is a schematic diagram illustrating an example of a configuration of a processing system of the fundus imaging apparatus according to the first modification example of the embodiments.

[FIG. 18] FIG. 18 is a flowchart illustrating an example of an operation of the fundus imaging apparatus according to the first modification example of the embodiments.

[FIG. 19] FIG. 19 is a schematic diagram illustrating an example of a configuration of an optical system of the fundus imaging apparatus according to a second modification example of the embodiments.

[FIG. 20] FIG. 20 is a schematic diagram illustrating an example of a configuration of an optical system of the fundus imaging apparatus according to a third modification example of the embodiments.

[MODES FOR CARRYING OUT THE INVENTION]

Referring now to the drawings, a fundus imaging apparatus is described in detail in some exemplary embodiments. Any of the contents of the documents cited in the present specification and arbitrary known techniques may be applied to the embodiments below.

A fundus imaging apparatus according to embodiments includes two or more curved mirrors, and is configured to scan a fundus of an eye to be examined with slit-shaped illumination light through the two or more curved mirrors and to receive returning light from the eye to be examined using an image sensor. The fundus imaging apparatus includes a slit in which an opening configured to be disposed at a fundus conjugate position is formed, and is configured to generate the slit-shaped illumination light by irradiating light from a light source onto the slit. Here, the fundus conjugate position is substantially conjugate optically to the fundus of the eye to be examined. In this case, at least one of both ends of the opening, which is formed in the slit, in a longitudinal direction is displaced in a shorter direction of the opening with reference to the longitudinal direction passing through a center of the opening. The image sensor is configured to be disposed at the fundus conjugate position.

Here, the "longitudinal direction" of the opening formed in the slit means a direction (longer direction) in which a long side of a rectangle that circumscribes the opening formed in the slit extends. The "shorter direction" of the opening formed in the slit means a direction (widthwise direction, shortitudinal direction) in which a short side of a rectangle that circumscribes the aperture formed in the slit extends.

In some embodiments, the opening formed in the slit includes a portion that changes in a curved shape from the center of the opening toward the at least one of the both ends. For example, the opening formed in the slit is configured to change in a curved shape from the center of the opening toward at least one of the both ends of the opening.

In some embodiments, the opening formed in the slit includes a portion that changes in a linear manner from the center of the opening toward the at least one of the both ends. For example, the opening formed in the slit is configured to change in a linear manner from the center of the opening toward the at least one of the both ends of the opening. In some embodiments, the opening formed in the slit includes a portion that changes in a curved shape from the center of the opening toward the at least one of the both ends and a potion that changes in a linear manner from the center of the opening toward the at least one of the both ends.

In some embodiments, each of the two or more curved mirrors has one or more focal points, and the two or more curved mirrors are arranged so as to share at least one of the two or more focal points. For example, the two or more curved mirrors are arranged so that the two or more focal points of the two or more curved mirrors are disposed on an approximately same plane (common plane of focal points).

For example, the fundus imaging apparatus is configured to illuminate the fundus with the illumination light so that a longitudinal direction of a slit image (image of the opening formed in the slit) formed by the illumination light projected onto the fundus is approximately parallel to a plane including the two or more focal points, and to scan the fundus with the illumination light in a direction intersecting the longitudinal direction (specifically, direction orthogonal to the longitudinal direction).

Examples of the curved mirror include an ellipsoidal mirror, a paraboloidal mirror, a hyperboloidal mirror, a free-form surface mirror, and a mirror whose reflective surface is represented by higher-order polynomials. The reflective surface of the curved mirror may be a concave-shaped reflective surface or a convex-shaped reflective surface. In other words, examples of the curved mirror include an ellipsoidal concave mirror, an ellipsoidal convex mirror, a paraboloidal concave mirror, a paraboloidal convex mirror, a hyperboloidal concave mirror, a hyperboloidal convex mirror, a free-form surface mirror having a concave-shaped reflective surface, a free-form surface mirror having a convex-shaped reflective surface, a concave mirror whose reflective surface is represented by higher-order polynomials, and a convex mirror whose reflective surface is represented by higher-order polynomials.

In the present specification, the focal point may include not only a fixed point uniquely determined by the shape of the curved surface, but also a position where the light beams (light fluxes) reflected on the reflective surface are more focused than at other positions.

The common plane of focal points is preferably a plane on which all focal points of the two or more curved mirrors are disposed. However, the common plane of focal points may be a plane on which two or more focal points, which are obtained by excluding at least one of all the focal points that the two or more curved mirrors have, are disposed.

The slit image projected onto the fundus is, for example, an image of the opening that has a constant slit width and changes in a curved shape or in a linear manner in the longitudinal direction from the center of the opening toward the end. However, the slit width does not have to be a constant width from the center of the opening toward the end.

The aberration of the slit image on the fundus caused by the curved mirror can be minimized for the direction orthogonal to the arrangement direction of the focal points with reference to the common plane of focal points including the two or more focal points, compared to the arrangement direction of the two or more focal points. This allows to minimize the separation between the slit image projected onto the fundus and the light receivable region (light-receptive region) of the returning light at the fundus conjugate position substantially conjugate optically to the fundus, without being affected by aberrations in the arrangement direction of the two or more focal points of the two or more curved mirrors that relay the slit-shaped illumination light. As a result, there is no longer a need to secure an excessively large region of the light receivable region (light-receptive region) and to include a region for the slit image and its separation. Since the light receivable region (light-receptive region) can be smaller, unnecessary light flux is not captured. Thereby, this allows to improve the contrast of fundus images and to reduce the occurrence of flare and/or ghost, without the need for advanced optical design. Therefore, higher-quality fundus images of the eye to be examined can be acquired.

A method of controlling the fundus imaging apparatus according to the embodiments includes one or more steps for realizing the processing executed by a processor (computer) in the fundus imaging apparatus according to the embodiments. A program according to the embodiments causes the processor to execute each step of the method of controlling the fundus imaging apparatus according to the embodiments. A recording medium (storage medium) according to the embodiments is a computer readable non-transitory recording medium (storage medium) on which the program according to the embodiments is recorded.

The term "processor" as used herein refers to a circuit such as, for example, a central processing unit (CPU), a graphics processing unit (GPU), an application specific integrated circuit (ASIC), and a programmable logic device (PLD). Examples of PLD include a simple programmable logic device (SPLD), a complex programmable logic device (CPLD), and a field programmable gate array (FPGA). The processor realizes, for example, the function according to the embodiments by reading out a computer program stored in a storage circuit or a storage device and executing the computer program.

Hereinafter, a case where the fundus imaging apparatus according to the embodiments includes two or more ellipsoidal concave mirrors as the two or more curved mirrors will be mainly described.

Hereinafter, for convenience of explanation, an optical axis direction of the illumination light incident on the eye to be examined is assumed to be a Z direction, a horizontal direction (left-right direction) orthogonal to the Z direction is assumed to be an X direction, and a vertical direction (up-down direction) orthogonal to the Z direction is assumed to be a Y direction. Furthermore, hereinafter, "longitudinal direction" of the slit image is assumed to be meant to be a direction in which the long side of the rectangle that circumscribes the slit image extends (longer direction), and "shorter direction" of the slit image is assumed to be meant to be a direction in which the short side of the rectangle circumscribed by the slit image extends (shortitudinal direction).

<Configuration>

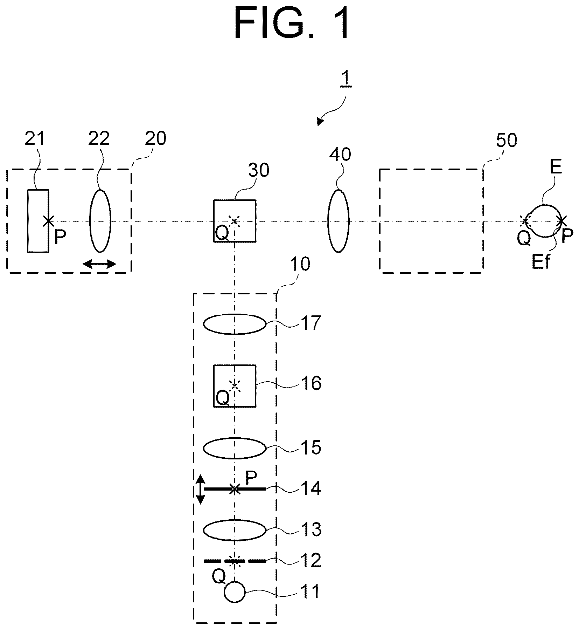

FIG. 1 shows an example of a configuration of an optical system of the fundus imaging apparatus according to the embodiments. It should be noted that, in FIG. 1, a position substantially conjugate optically to the fundus of the eye to be examined is illustrated as a fundus conjugate position P, and a position substantially conjugate optically to an iris (pupil) of the eye to be examined is illustrated as an iris conjugate position (pupil conjugate position) Q.

A fundus imaging apparatus 1 according to the embodiments includes an illumination optical system 10, an imaging optical system 20, a hole mirror 30 as an optical path dividing member, a relay lens 40, and a relay optical system 50. In some embodiments, the relay optical system 50 includes the relay lens 40.

(Illumination optical system 10)

The illumination optical system 10 is configured to generate slit-shaped illumination light (illumination light whose luminous flux cross-sectional shape is slit-shaped), and to project the generated illumination light onto the hole mirror 30, as a slit projection optical system. The illumination optical system 10 includes an illumination light source 11, an iris aperture 12, a relay lens 13, a slit 14, a relay lens 15, an optical scanner 16, and a relay lens 17. In some embodiments, at least one of the relay lenses 13, 15, or 17 includes one or more lenses.

The illumination light source 11 includes a visible light source that generates light in the visible region. For example, the illumination light source 11 generates light having a central wavelength in the wavelength range of 420 nm to 700 nm. This type of illumination light source 11 includes, for example, an LED (Light Emitting Diode), an LD (Laser Diode), a halogen lamp, or a xenon lamp. In some embodiments, the illumination light source 11 includes a white light source or a light source capable of outputting light with each color component of RGB. In some embodiments, the illumination light source 11 includes a light source capable of switching to output the light in infrared region or the light in visible region.

The iris aperture 12 (specifically, its opening(s) (its aperture(s))) can be disposed at the iris conjugate position Q. The iris aperture position Q is a position conjugate optically to the iris (pupil) of the eye E to be examined, or the vicinity of this position. In the iris aperture 12, one or more openings are formed at eccentric position(s) from an optical axis of an optical path of light output from the illumination light source 11. The opening formed in the iris aperture 12 defines an incident position (incident shape) of the illumination light on the iris of the eye E to be examined.

In some embodiments, a relative position between the illumination light source 11 and the opening formed in the iris aperture 12 is configured to be changeable. Thereby, the light quantity distribution of the light passing through the opening formed in the iris aperture 12 can be changed.

In some embodiments, the illumination light source 11 has a function of the iris aperture 12. In this case, a member that realizes the function of the iris aperture 12 in the illumination light source 11 is disposed at the iris conjugate position Q.

The slit 14 (specifically, its opening(s) (its aperture(s))) can be disposed at the fundus conjugate position P. The fundus conjugate position P is a position substantially conjugate optically to the fundus Ef of the eye E to be examined, or the vicinity of this position. The opening formed in the slit 14 defines a shape of an illumination region (irradiated pattern shape) on the fundus Ef of the eye E to be examined. The illumination light emitted from the illumination light source 11 passes through the slit 14 and is projected onto the fundus Ef as slit-shaped illumination light.

The slit 14 is movable in the optical axis direction of the illumination optical system using a movement mechanism (not show) (specifically, movement mechanism 14D described below). This allows to move the position of the slit 14 in accordance with the state of the eye E to be examined (specifically, the dioptric power (refractive power) or the shape of the fundus Ef).

In some embodiments, the slit 14 is configured so that at least one of the position of the opening or the shape of the opening can be changed in accordance with the state of the eye E to be examined without being moved in the optical axis direction. The function of the slit 14 with these functions is, for example, realized by a liquid crystal shutter.

The optical scanner 16 (specifically, its deflection surface) can be disposed at the iris conjugate position Q, that is a position conjugate optically to the iris (pupil) of the eye E to be examined or the vicinity of the position. The optical scanner 16 is a uniaxial optical scanner that deflects an orientation of the deflection surface in a predetermined deflection direction. The optical scanner 16 one-dimensionally deflects the slit-shaped illumination light formed by passing through the slit 14. The optical scanner 16 deflects the illumination light in a direction intersecting (specifically, orthogonal to) the longitudinal direction of the slit image formed by the slit-shaped illumination light projected onto the fundus Ef. Thereby, the slit image moves in the direction intersecting the longitudinal direction of the slit image (scan direction).

The optical scanner 16 includes, for example, a galvanometer scanner, a MEMS (Micro Electro Mechanical System) scanner, a polygon mirror, or a resonant scanner. For example , the optical scanner 16 includes the galvanometer scanner that deflects the illumination light within a predetermined deflection angular range with reference to a predetermined deflection direction.

In some embodiments, the optical scanner 16 is a biaxial optical scanner that two-dimensionally deflects the slit-shaped illumination light. For example, the optical scanner 16 includes a first scanner and a second scanner. The first scanner deflects the illumination light so as to move the illumination region on the fundus Ef in a horizontal direction orthogonal to the optical axis of the illumination optical system 10. The second scanner deflects the illumination light deflected by the first scanner so as to move the illumination region on the fundus Ef in a vertical direction orthogonal to the optical axis of the illumination optical system 10.

The light from the illumination light source 11 that has passed through the opening(s) formed in the iris aperture 12 is transmitted through the relay lens 13, passes through the opening(s) formed in the slit 14, and is output as the slit-shaped illumination light. The slit-shaped illumination light emitted from the slit 14 is transmitted through the relay lens 15, is deflected one-dimensionally by the optical scanner 16, is transmitted through the relay lens 17, and is guided to the hole mirror 30.

In some embodiments, the illumination optical system 10 includes a projector with a light source, and the projector emits the slit-shaped illumination light. In this case, the projector is provided instead of the illumination light source 11, the iris aperture 12, the relay lens 13, and the slit 14 that are in FIG. 1. Examples of the projector include an LCD (Liquid Crystal Display) type projector using a transmissive LCD panel, an LCOS (Liquid Crystal On Silicon) type projector using a reflective LCD panel, a DLP (Digital Light Processing) (registered trademark) type projector using a DMD (Digital Mirror Device).

(Hole mirror 30)

The hole mirror 30 (specifically, its hole part described below) can be disposed at the iris conjugate position Q. The hole mirror 30 is an optical path dividing member that spatially divides the optical path of the illumination light and the optical path of the returning light from the eye E to be examined, onto which the illumination light has been irradiated.

In the hole mirror 30, the hole part, through which the optical axis passes, is formed in a central part of a base substance, and a reflective surface, which reflects the light, is provided around a peripheral part of the central part. In FIG. 1, the hole mirror 30, which is positioned so that the optical axis of the imaging optical system 20 passes through the hole part, is configured to reflect the illumination light from the illumination optical system 10 on the reflective surface provided in the peripheral part to guide the reflected illumination light to the eye E to be examined. In some embodiments, the hole mirror 30, which is positioned so that the optical axis of the illumination optical system 10 passes through the hole part, is configured to reflect the returning light from the eye E to be examined on the reflective surface provided in the peripheral part to guide the reflected returning light to the imaging optical system 20. In some embodiments, in the hole mirror 30, the hole part, through which the optical axis passes, is formed in one side of the base substance, and the reflective surface, which reflects the light, is provided in the other side of the base substance. In this case, the light passing through the hole part is guided to the imaging optical system 20, and the light reflected on the reflective surface is guided to the eye E to be examined. Alternatively, conversely, the light passing through the hole part is guided to the eye E to be examined, and the light reflected on the reflective surface is guided to the imaging optical system 20.

The illumination light from the illumination optical system 10 is reflected on the reflective surface on the peripheral part of the hole part formed in the hole mirror 30, and is guided to the relay lens 40. The returning light from the eye E to be examined is transmitted through the relay lens 40, passes through the hole part formed in the hole mirror 30, and is guided to the imaging optical system 20.

In some embodiments, a beam splitter, a half mirror, a multifaceted mirror, or a dichroic mirror is placed instead of the hole mirror 30.

(Imaging optical system 20)

The imaging optical system 20 is configured to receive the returning light from the eye E to be examined, which has passed through the hole part formed in the hole mirror 30, as a slit light receiving optical system. Here, the returning light from the eye E to be examined is scattered light (reflected light) of the illumination light incident on the eye E to be examined. In some embodiments, the returning light from the eye E to be examined includes scattered light (reflected light) of the illumination light incident on the eye E to be examined and fluorescence and its scattered light using the illumination light incident on the eye E to be examined as excitation light.

The imaging optical system 20 includes an image sensor 21 and a focusing lens 22.

The focusing lens 22 is movable in the optical axis direction of the imaging optical system 20 using a movement mechanism (not show) (specifically, movement mechanism 22D described below). This allows to adapt the state of the eye E to be examined and to image the returning light from the eye E to be examined on the light receiving surface of the image sensor 21.

The image sensor 21 realizes the function of the two-dimensional image sensor as a pixelated photodetector. The light receiving surface (detecting surface, imaging surface) of the image sensor 21 can be disposed at the fundus conjugate position P. The image sensor 21 is configured to be capable of setting a light receivable region (light-receptive region) that can be virtually moved at the fundus conjugate position P.

For example, the light receiving result(s) acquired by the image sensor 21 is/are captured and read out using a rolling shutter system (method). In some embodiments, the light receiving result(s) acquired by the image sensor 21 is/are captured and read out using a global shutter system (method) in which the light receivable region can be changed or be moved. In some embodiments, a controller described below performs readout control of the light receiving result(s) by controlling the image sensor 21. In some embodiments, the image sensor 21 can automatically output the light receiving result(s) for a predetermined number of lines, along with information indicating the light receiving position(s).

The image sensor 21 with such a configuration includes a CMOS (Complementary metal oxide semiconductor) image sensor. In this case, the image sensor 21 includes a plurality of pixels (light receiving elements). The plurality of pixels includes a plurality of pixel groups arranged in a column direction. Each of the plurality of pixel groups includes pixels arranged in a row direction. Specifically, the image sensor 21 includes a plurality of pixels arranged two-dimensionally, a plurality of vertical signal lines, and a horizontal signal line.

In some embodiments, the image sensor 21 is a CCD (Charge Coupled Device) image sensor, for example.

By capturing (reading out) the light receiving result(s) of the returning light using the rolling shutter system for such an image sensor 21, the image in the light receivable region corresponding to a desired virtual opening shape extending in the row direction is acquired. Such control is disclosed in, for example, Patent Document 2 or U.S. Patent No. 8,237,835.

The returning light from the eye E to be examined, onto which the illumination light from the illumination optical system 10 has irradiated, passes through the hole part formed in the hole mirror 30, is transmitted through the focusing lens 22, and forms an image on the light receiving surface of the image sensor 21.

In some embodiments, one or more relay lenses are disposed between the focusing lens 22 and the image sensor 21.

(Relay optical system 50)

The relay optical system 50 is disposed between the relay lens 40 and the eye E to be examined. The relay optical system 50 is configured to relay the slit image, which is formed by the slit-shaped illumination light generated by the illumination optical system 10, to the fundus Ef of the eye E to be examined. The relay optical system 50 includes a reflective optical system with an angle-of-view extension function. In this case, the relay optical system 50 may further include a refractive optical system such as an aberration correcting lens. In some embodiments, the relay optical system 50 does not have the angle-of-view extension function.

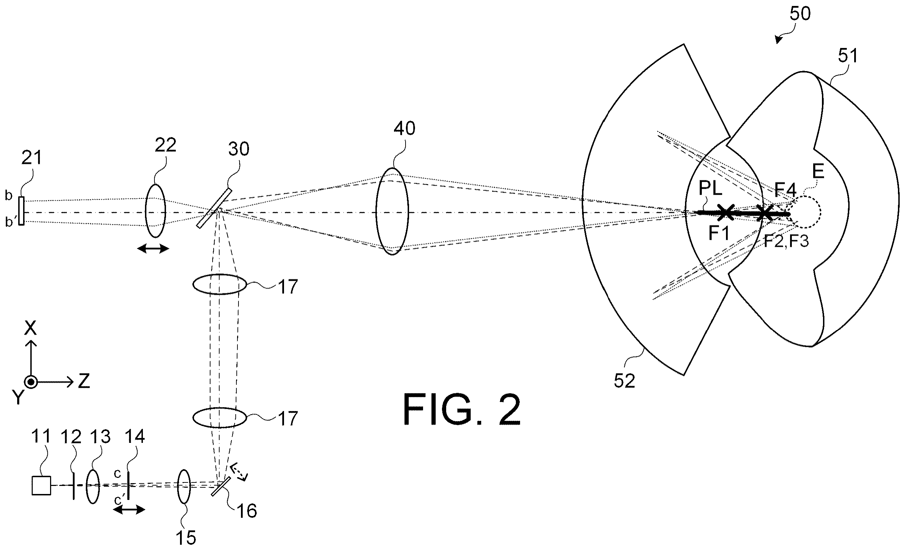

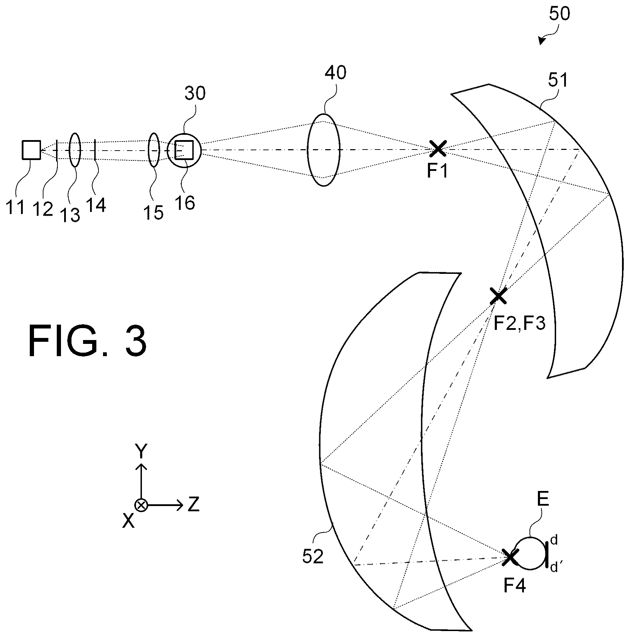

FIG. 2 and FIG. 3 show examples of a configuration of the relay optical system 50 in FIG. 1. FIG. 2 schematically represents an example of the configuration of the relay optical system 50 when viewed from the top (X-Z plane). FIG. 3 schematically represents an example of the configuration of the relay optical system 50 when viewed from the side (Y-Z plane). In FIG. 2 and FIG. 3, like parts are designated by like reference numerals as in FIG. 1 and repetitious description of such parts may not be provided.

As shown in FIG. 2 and FIG. 3, the relay optical system 50 includes a first ellipsoidal concave mirror 51 and a second ellipsoidal concave mirror 52.

(First ellipsoidal concave mirror 51)

A reflective surface of the first ellipsoidal concave mirror 51 is a concave-shaped ellipsoid. The first ellipsoidal concave mirror 51 is an example of the curved mirror or the concave mirror.

The first ellipsoidal concave mirror 51 has two optically conjugate focal points (first focal point F1, second focal point F2). The relay lens 40 is positioned so that the iris conjugate position (pupil conjugate position) Q substantially coincides with the first focal point F1.

(Second ellipsoidal concave mirror 52)

A reflective surface of the second ellipsoidal concave mirror 52 is a concave-shaped elliptical surface. The second ellipsoidal concave mirror 52 is an example of the curved mirror or the concave mirror.

The second ellipsoidal concave mirror 52 has two optically conjugate focal points (first focal point F3, second focal point F4). The second ellipsoidal concave mirror 52 is positioned so that the first focal point F3 substantially coincides with the second focal point F2 of the first ellipsoidal concave mirror 51. In some embodiments, the second ellipsoidal concave mirror 52 is positioned so that the first focal point F3 substantially coincides with a position conjugate optically to the second focal point F2 of the first ellipsoidal concave mirror 51 (conjugate position of the second focal point F2) or the vicinity of the position conjugate optically to the second focal point F2. The eye E to be examined (iris) is positioned at the second focal point F4 of the second ellipsoidal concave mirror 52. In other words, the second ellipsoidal concave mirror 52 is positioned so that the second focal point F4 substantially coincides with an eye to be examined position where the eye E to be examined is positioned.

Furthermore, as shown in FIG. 2, the first ellipsoidal concave mirror 51 and the second ellipsoidal concave mirror 52 are positioned so that the two focal points (first focal point F1, second focal point F2) of the first ellipsoidal concave mirror 51 and the two focal points (first focal point F3, second focal point F4) of the second ellipsoidal concave mirror 52 are positioned on substantially the same plane (common plane PL of focal points). In addition, the slit 14 is disposed so that the longitudinal direction of the slit image formed by the slit-shaped illumination light projected onto the fundus Ef is approximately parallel to the common plane PL of focal points. In some embodiments, the slit 14 is disposed so that the longitudinal direction of the slit image formed by the slit-shaped illumination light projected onto the fundus Ef is positioned on the common plane PL of focal points, in a state where the position matching has been performed.

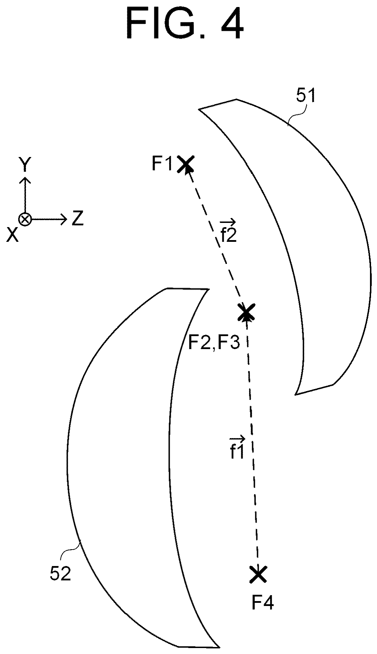

FIG. 4 schematically shows a relationship between the longitudinal direction of the slit image and the focal points of the first ellipsoidal concave mirror 51 and the second ellipsoidal concave mirror 52 that are shown in FIG. 3. In FIG. 4, like reference numerals designate like parts as in FIG. 3, and the redundant explanation may be omitted as appropriate.

Here, the longitudinal direction of the slit image on the fundus Ef is denoted as a vector "LV", and the direction orthogonal (perpendicular) to the longitudinal direction of the slit image is denoted as a vector "S". Further, a vector from the second focal point F4 toward the first focal point F3 of the second ellipsoidal concave mirror 52 is denoted as a vector "f1", and a vector from the second focal point F2 toward the first focal point F1 of the first ellipsoidal concave mirror 51 is denoted as a vector "f2". When denoting the normal vector of the common plane PL of focal points as a vector "n", the vector "n" is expressed as in Equation (1). In this case, the longitudinal direction of the slit image and the focal points of the first ellipsoidal concave mirror 51 and the second ellipsoidal concave mirror 52 are arranged so that the vectors "S" described above and the vector "n" are parallel to each other, as shown in Equation (2).

[Equation 1]

$$\vec{n} = \overrightarrow{f1} \times \overrightarrow{f2} \tag{1}$$

$$\vec{S} \parallel \vec{n} \tag{2}$$

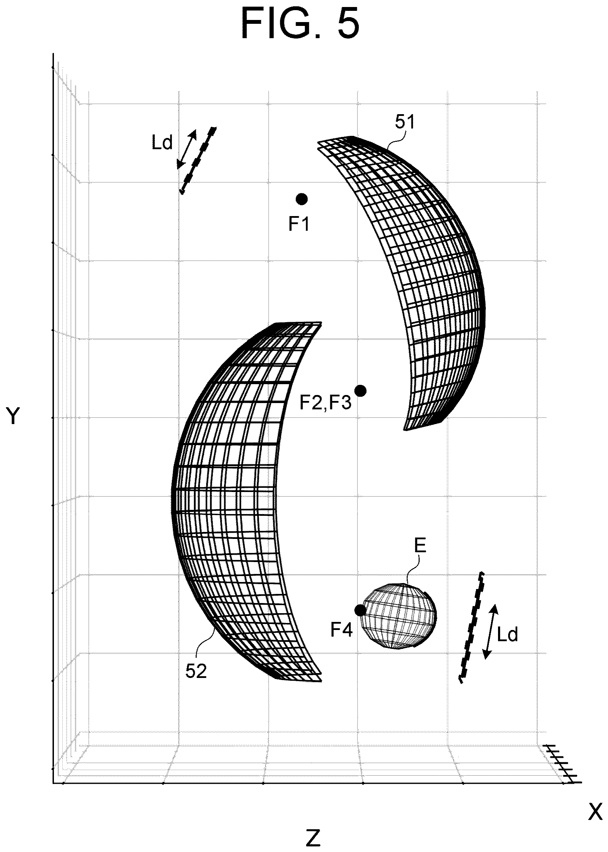

FIG. 5, FIG. 6, FIG. 7, and FIG. 8 show a relationship among the focal points of the first ellipsoidal concave mirror 51 according to the embodiments, the focal points of the second ellipsoidal concave mirror 52 according to the embodiments, and the slit image projected onto the fundus Ef. FIG. 5, FIG. 6, FIG. 7, and FIG. 8 represent wireframes indicating an example of a three-dimensional positional relationship among the first ellipsoidal concave mirror 51, the second ellipsoidal concave mirror 52, and the eye E to be examined. In FIG. 5, FIG. 6, FIG. 7, and FIG. 8, the longitudinal direction (Ld) of the slit image incident on the first ellipsoidal concave mirror 51, and the longitudinal direction (Ld) of the slit image projected onto the fundus Ef of the eye E to be examined are illustrated.

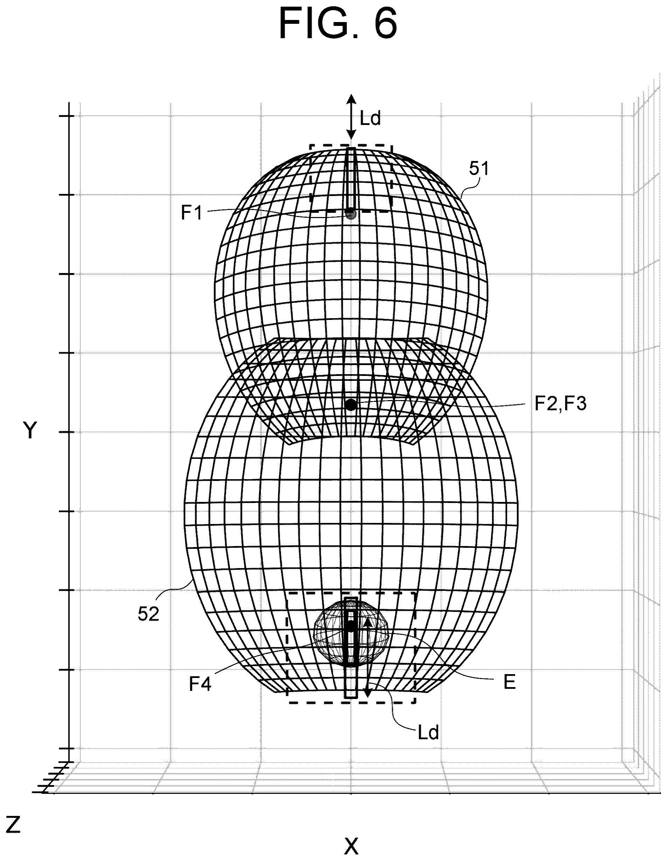

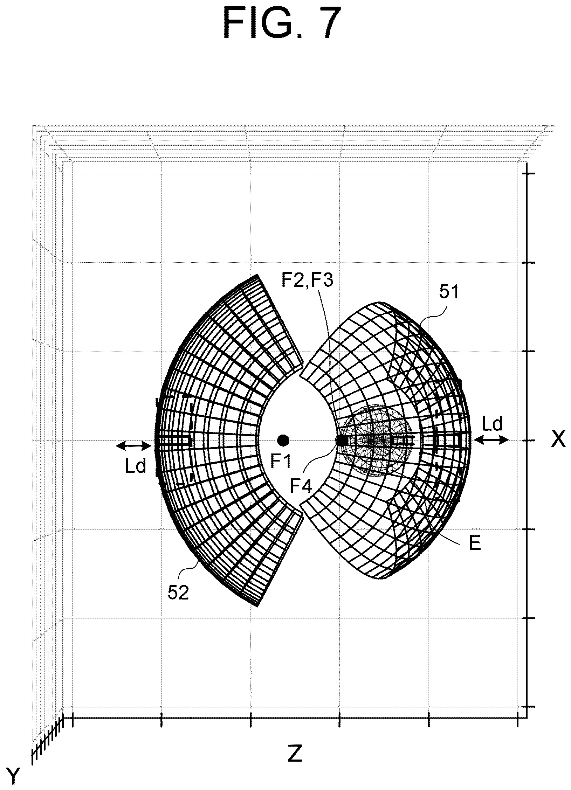

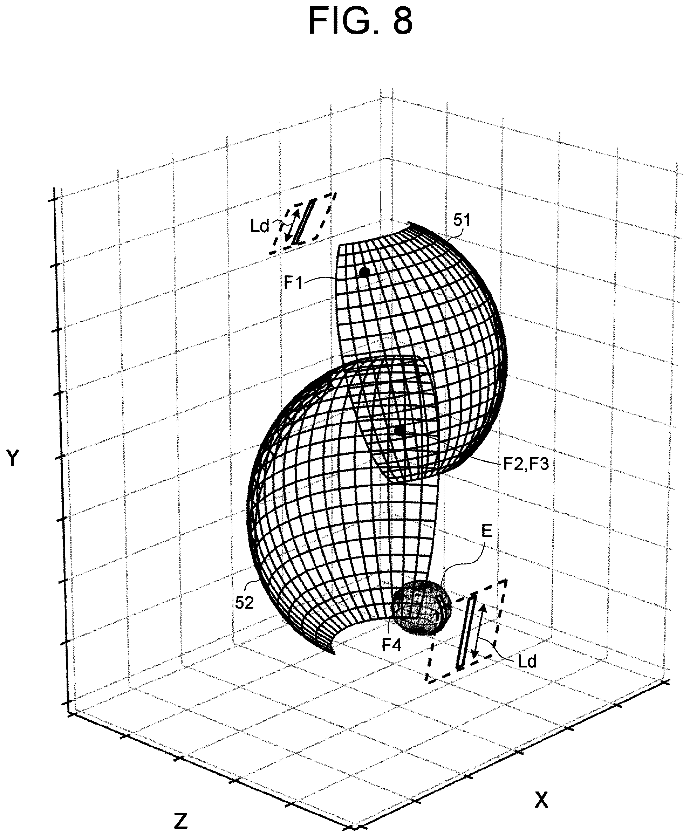

FIG. 5 represents a wireframe indicating a relationship among the first ellipsoidal concave mirror 51, the second ellipsoidal concave mirror 52, and the longitudinal direction Ld of the slit image, when viewed from the lateral (X) direction. FIG. 6 represents a wireframe indicating a relationship among the first ellipsoidal concave mirror 51, the second ellipsoidal concave mirror 52, and the longitudinal direction Ld of the slit image, when viewed from a side facing the reflective surface of the second ellipsoidal concave mirror 52. FIG. 7 represents a wireframe indicating a relationship among the first ellipsoidal concave mirror 51, the second ellipsoidal concave mirror 52, and the longitudinal direction Ld of the slit image, when viewed from the upper (top) direction (Y direction). FIG. 8 represents a wireframe indicating a relationship among the first ellipsoidal concave mirror 51, the second ellipsoidal concave mirror 52, and the longitudinal direction Ld of the slit image, when viewed from an oblique direction. In FIG. 5, FIG. 6, FIG. 7, and FIG. 8, like reference numerals designate like parts as in FIG. 2 and FIG. 3. The same description may not be repeated.

For example, as shown in FIG. 2, the first focal point F1 of the first ellipsoidal concave mirror 51, the second focal point F2 of the first ellipsoidal concave mirror 51, the first focal point F3 of the second ellipsoidal concave mirror 52, and the second focal point F4 of the second ellipsoidal concave mirror 52 are positioned on the same plane (common plane PL of focal points). As described above, the illumination optical system 10 projects the slit image, whose longitudinal direction is parallel to the common plane of focal points described above, onto the fundus Ef of the eye E to be examined, by entering the slit-shaped illumination light into the first ellipsoidal concave mirror 51 so that the longitudinal direction Ld of the slit image is approximately parallel to the common plane of focal points described above. More specifically, the illumination optical system 10 projects the slit image, whose longitudinal direction Ld is parallel to the common plane of focal points described above, onto the fundus Ef of the eye E to be examined, by entering the slit-shaped illumination light, in which the longitudinal direction Ld of the slit image is arranged on the common plane of focal points described above. The illumination optical system 10 scans the fundus Ef with the illumination light by scanning this slit image in the direction orthogonal to the longitudinal direction Ld using the optical scanner 16.

In the configuration as described above, the slit-shaped illumination light from the illumination optical system 10 is reflected on the reflective surface of the first ellipsoidal concave mirror 51, and is guided to the second focal point F2 of the first ellipsoidal concave mirror 51. The illumination light that has been guided to the second focal point F2 (first focal point F3) is guided to the reflective surface of the second ellipsoidal concave mirror 52, is reflected on this reflective surface, and is guided to the eye E to be examined arranged at the second focal point F4 of the second ellipsoidal concave mirror 52.

The illumination light that has been guided to the eye E to be examined enters the eye through the pupil and is irradiated onto the fundus Ef. The returning light from the eye E to be examined is emitted outside of the eye E to be examined through the pupil, travels in the same path as the outward path in the opposite direction, and is guided to the first focal point F1 of the first ellipsoidal concave mirror 51. The returning light that has been guided to the first focal point F1 passes through the hole part formed in the hole mirror 30, and is guided to the imaging optical system 20, as described above. In the imaging optical system 20, the returning light is received on the light receiving surface of the image sensor 21, and the light receiving results of the returning light in the light receivable region set on the light receiving surface are captured.

In the fundus imaging apparatus 1 with such a configuration, in order to reduce flare from anterior segment of the eye E to be examined, it is desirable that the illumination light flux and the light receiving light flux (imaging light flux) are separated at near the iris of the eye E to be examined (near the second focal point F4 of the second ellipsoidal concave mirror 52). In this case, at least one of a light flux passage region of the illumination optical system 10, through which the illumination light flux passes, and a light flux passage region of the imaging optical system 20, through which the light receiving light flux passes, no longer coincides with the second focal point F4 of the second ellipsoidal concave mirror 52.

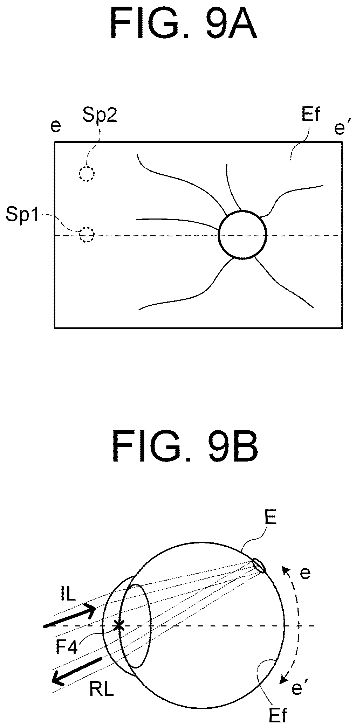

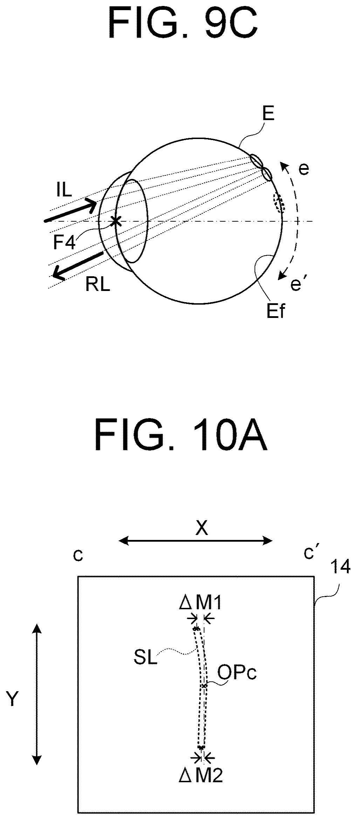

FIG. 9A, FIG. 9B, and FIG. 9C show explanatory diagrams of an illumination light flux and a light receiving light flux according to a comparative example of the embodiments. FIG. 9A schematically represents irradiated positions Sp1 and Sp2 of the illumination light flux on the fundus Ef. FIG. 9B schematically represents an illumination light flux IL and a light receiving light flux RL at the irradiated positions on the fundus Ef. FIG. 9C schematically represents the illumination light flux IL and the light receiving light flux RL at the irradiated position Sp2 on the fundus Ef. In FIG. 9A, FIG. 9B, and FIG. 9C, like reference numerals designate like parts as in FIGs. 1 to 8. The same description may not be repeated.

At the irradiated position Sp1 on the fundus Ef shown in FIG. 9A, as shown in FIG. 9B, the illumination light flux IL, which enters into the eye, and the light receiving light flux RL, which emits from the eye, coincide on the fundus Ef. In contrast, at the irradiated position Sp2 on the fundus Ef shown in FIG. 9A, as shown in FIG. 9C, the illumination light flux IL, which enters into the eye, and the light receiving light flux RL, which emits from the eye, does not coincide on the fundus Ef. As a result, differences between an image formed by the light receiving light flux RL at the irradiated position Sp1 and an image formed by the light receiving light flux RL at the irradiated position Sp2 are appeared. Therefore, distortion occurs in the slit image formed on the light receiving surface of the image sensor 21. This means that the distortion of the slit image changes significantly as the irradiated position of the illumination light flux at the iris of the eye E to be examined moves away from the second focal point F4. In this case, the slit image does not fit within the light receivable region (exposure region) that is preset as a virtual opening range on the image sensor 21, which causes deterioration in image quality.

Therefore, in the present embodiment, without complicating the readout control of the image sensor 21, the shape of the opening formed in the slit 14 is deformed in accordance with the shape of the light receivable region preset on the image sensor 21.

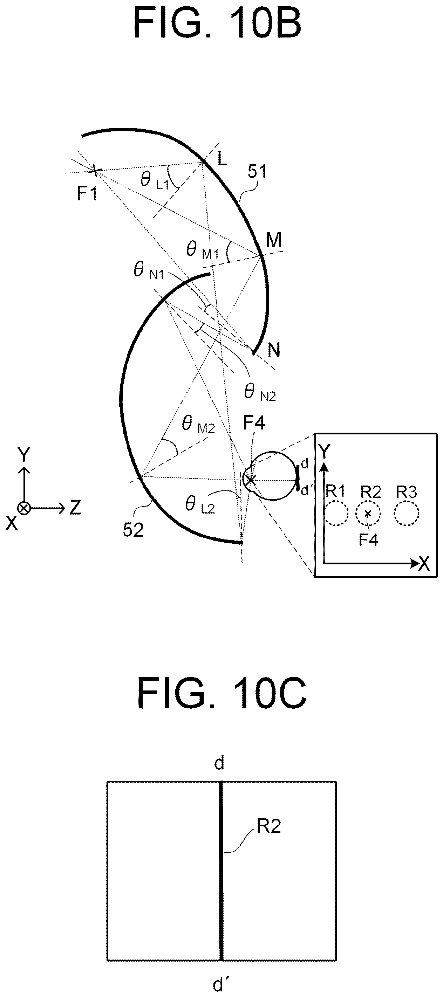

FIGs. 10A to 10E show schematic diagrams for explaining the shape of the opening formed in the slit 14 according to the embodiments. FIG. 10A schematically represents an opening SL formed in the slit 14. FIG. 10B schematically represents the light flux passage region of the illumination light flux at the iris of the eye E to be examined. FIGs. 10C to 10E schematically represent the slit image projected onto the fundus Ef according to the light flux passage region paths of the illumination light flux in FIG. 10B.

Light from the illumination light source 11 that has passed through the opening SL shown in FIG. 10A is emitted as the slit-shaped illumination light whose longitudinal direction follows the direction orthogonal to the c-c' direction in FIG. 2. One end of both ends of the opening SL, which is formed in the slit 14, in the longitudinal direction is displaced by $\Delta M1$ in a shorter direction (X direction in FIG. 10A) of the opening SL with reference to the longitudinal direction (Y direction in FIG. 10A) passing through a center OPc of the opening. The other end of the both ends of the opening SL in the longitudinal direction is displaced by $\Delta M2$ in the shorter direction of the opening SL with reference to the longitudinal direction passing through the center OPc of the opening. Here, the center OPc of the opening corresponds to a center position in the shorter direction that defines the width of the opening SL at the center of the opening SL in the longitudinal direction. In other words, in the slit 14, the opening SL is formed so as to change in a curved manner in the longitudinal direction from the center OPc of the opening toward the both ends. In FIG. 10A, the opening SL includes a portion that changes in a curved shape from the center OPc of the opening toward at least one of the both ends.

In FIGs. 10B to 10E, the shape of the opening SL formed in the slit 14 is assumed to be a rectangular shape extending in the longitudinal direction.

When the position of the light flux passage region of the illumination light flux is at the second focal point F4 of the second ellipsoidal concave mirror 52, a slit image (R2) extending in the longitudinal direction is projected (the ends of the slit image are not displaced in the shorter direction), as shown in FIG. 10C.

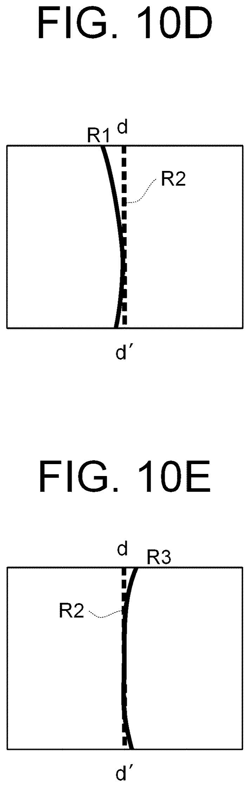

In contrast, when the position of the light flux passage region of the illumination light flux is displaced from the second focal point F4 of the second ellipsoidal concave mirror 52, slit images (R1, R3) extending in the longitudinal direction are projected, as shown in FIG. 10D and FIG. 10E. The ends of these slit images are displaced in the shorter direction. In other words, as the light flux passage region of the illumination light flux moves away from the second focal point F4 of the second ellipsoidal concave mirror 52, the ends of the slit image, which should not originally be displaced in the shorter direction, are displaced in the shorter direction.

On the other hand, at least one of the both ends of the opening SL formed in the slit 14 according to the embodiments is displaced in accordance with a maximum value of an absolute value of a difference between an incidence angle of the illumination light on the first ellipsoidal concave mirror 51 and an incidence angle of the illumination light on the second ellipsoidal concave mirror 52 within an irradiation range of the illumination light on the first ellipsoidal concave mirror 51 and the second ellipsoidal concave mirror 52.

Hereinafter, the displacement of the both ends of the opening SL formed in the slit 14 will be described. When an ellipsoidal concave mirror is tilted so as to increase the angular magnification, the incidence angle of the illumination light entering the eye varies greatly depending on the incident position of the illumination light on the reflective surface of the ellipsoidal concave mirror. In this case, an asymmetric distortion occurs in the slit image relative to the optical axis (light flux passing through the incident position M in FIG. 10B).

Here, the incidence angle of the illumination light entering the incident position L on the reflective surface of the first ellipsoidal concave mirror 51 is denoted as $\theta_{L1}$, and the incidence angle of the illumination light on the reflective surface of the second ellipsoidal concave mirror 52 is denoted as $\theta_{L2}$. Further, the incidence angle of the illumination light entering the incident position M on the reflective surface of the first ellipsoidal concave mirror 51 is denoted as $\theta_{M1}$, and the incidence angle of the illumination light on the reflective surface of the second ellipsoidal concave mirror 52 is denoted as $\theta_{M2}$. Furthermore, the incidence angle of the illumination light entering the incident position N on the reflective surface of the first ellipsoidal concave mirror 51 is denoted as $\theta_{N1}$, and the incidence angle of the illumination light on the reflective surface of the second ellipsoidal concave mirror 52 is denoted as $\theta_{N2}$.

For each light flux of the illumination light, the difference $\Delta\theta$ between the incidence angle of the first ellipsoidal concave mirror 51 and the incidence angle of the second ellipsoidal concave mirror 52 varies depending on the incident position of the illumination light on the reflective surface of the first ellipsoidal concave mirror 51. For the light flux passing through the incident position L, the incidence angle difference $\Delta\theta_L = |\theta_{L1} - \theta_{L2}|$. For the light flux passing through the incident position M, the incidence angle difference $\Delta\theta_M = |\theta_{M1} - \theta_{M2}|$. For the light flux passing through the incident position M, the incidence angle difference $\Delta\theta_N = |\theta_{N1} - \theta_{N2}|$. Here, $\Delta\theta_L > \Delta\theta_M > \Delta\theta_N$.

The greater the difference $\Delta\theta$ between the incidence angles of the first ellipsoidal concave mirror 51 and the second ellipsoidal concave mirror 52 becomes, as in the light flux passing through the incident position L in FIG. 10B, the greater the deviation from the slit image when passing through the incident position M in FIG. 10B becomes (i.e., the more the curve increases). In contrast, the smaller the difference $\Delta\theta$ between the incidence angles of the first ellipsoidal concave mirror 51 and the second ellipsoidal concave mirror 52 becomes, as in the light flux passing through the incident position N in FIG. 10B, the smaller the deviation from the slit image when passing through the incident position M in FIG. 10B becomes (i.e., the less the curve becomes).

Therefore, by displacing at least one of the ends of the opening SL formed in the slit 14 in accordance with the maximum value of the absolute value of the difference between the incidence angle of the illumination light on the first ellipsoidal concave mirror 51 and the incidence angle of the illumination light on the second ellipsoidal concave mirror 52, the deviation from the slit image projected onto the fundus Ef can be eliminated.

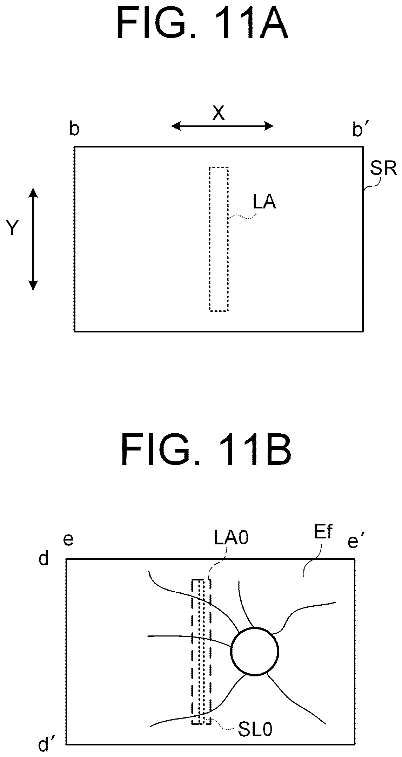

FIG. 11A and FIG. 11B schematically show the slit-shaped illumination light and the light receivable region on the image sensor 21. FIG. 11A schematically represents a light receivable region LA on the light receiving surface SR of the image sensor 21. FIG. 11B schematically represents a slit image SL0 projected onto the fundus Ef and a light receivable region LA0 on the light receiving surface of the image sensor 21.

The slit-shaped illumination light, which is generated by passing through the opening(s) formed in the slit 14, is deflected by the optical scanner 16, and enters the eye through the pupil of the eye E to be examined through the first ellipsoidal concave mirror 51 and the second ellipsoidal concave mirror 52, and is projected onto the fundus Ef, as described above. The optical scanner 16 scans so that the illumination region defined by the slit image SL0 moves in a direction orthogonal to the longitudinal direction (d-d' direction in FIG. 3) of the slit image SL0 on the fundus Ef , as shown in FIG. 11B.

As shown in FIG. 11A, the image sensor 21 is configured to be capable of virtually moving the light receivable region LA on the light receiving surface SR. Thereby, the reduction of contrast, the occurrence of flare and/or ghost, which are caused by the reception of unnecessary light from the eye E to be examined, can be prevented. The image sensor 21 changes the position of the light receivable region LA on the light receiving surface SR in synchronization with the movement of the illumination region of the illumination light on the fundus Ef. Specifically, as shown in FIG. 11B, the light receivable region LA of the image sensor 21 is set in accordance with the light receivable region LA0 on the fundus Ef so as to include a range of the slit image SL0 on the fundus Ef. In other words, the light receivable region LA is set in a strip shape whose longitudinal direction follows a direction orthogonal to the b-b' direction in FIG. 2. And, the image sensor 21 sequentially moves the light receivable region LA on the light receiving surface SR in accordance with the light receivable region LA0, in synchronization with the movement of the slit image SL0 on the fundus Ef so as to include the range of the slit image SL0.

Idealistically, it is desirable that the slit image SL0 and the light receivable region LA0 coincide with each other on the fundus Ef. However, due to aberrations in the optical system, the light receivable region LA0 must be wider than the slit image SL0.

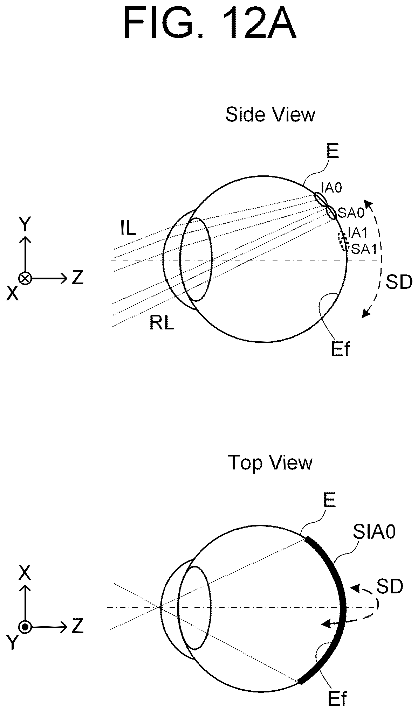

FIG. 12A shows a diagram for explaining an operation of a fundus imaging apparatus according to a comparative example of the embodiments. FIG. 12A schematically represents a side view (Side View) of the eye E to be examined scanned using the slit-shaped illumination light when viewed from the X direction, and a top view (Top View) of the eye E to be examined scanned using the slit-shaped illumination light when viewed from the Y direction.

In the comparative example of the embodiments, the longitudinal direction of the slit image is assumed to be orthogonal to the common plane of focal points described above, and the fundus Ef is assumed to be scanned in the direction perpendicular to the longitudinal direction of the slit image (in other words, the common plane of focal points described above is assumed to be parallel to or coincide with the scan direction SD).

In the present comparative example, the slit image SIA0 projected onto the fundus Ef is moved along the scan direction SD. In this case, the illumination region IA0 (IA1) illuminated by the illumination light flux IL and the light-receptive region SA0 (SA1) on the light receiving surface of image sensor 21 are separated as they move away from the optical axis, due to the aberration of the optical system. Therefore, in order to suppress the decrease in the light receiving amount of the light receiving light flux RL from the light-receptive region SA0, the light receivable region LA0 in FIG. 11B must be wider than the slit image SL0.

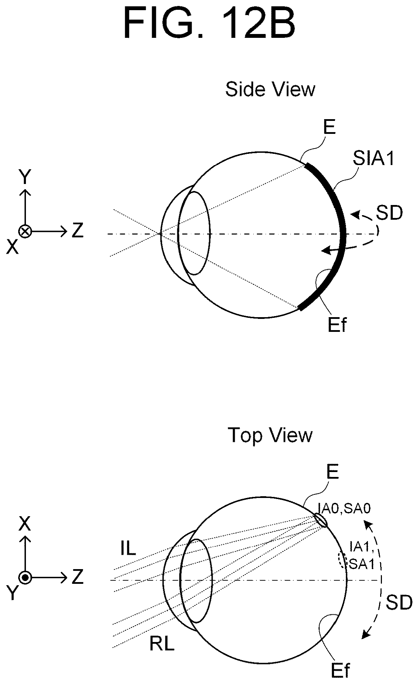

FIG. 12B shows a diagram for explaining an operation of the fundus imaging apparatus 1 according to the embodiments. FIG. 12B schematically represents a side view (Side View) of the eye E to be examined scanned using the slit-shaped illumination light when viewed from the X direction, and a top view (Top View) of the eye E to be examined scanned using the slit-shaped illumination light when viewed from the Y direction, in the same way as in FIG. 12A.

In the embodiments, as described above, the longitudinal direction of the slit image coincides with or is approximately parallel to a direction the common plane of focal points described above, and the fundus Ef is scanned in the direction orthogonal to the longitudinal direction of the slit image.

In other words, in the embodiments, the slit image SIA1 projected onto the fundus Ef is moved along the scan direction SD. The aberration of the slit image on the fundus caused by the curved mirror such as an ellipsoidal concave mirror can be minimized for the direction orthogonal to the arrangement direction of the focal points with reference to the common plane of focal points including the two or more focal points, compared to the arrangement direction of the two or more focal points. Therefore, the separation between the illumination region IA0 (IA1) illuminated by the illumination light flux IL and the light-receptive region SA0 (SA1) can be minimized, even when they are far from the optical axis. Therefore, there is no longer a need to suppress the decrease in the light receiving amount of the light receiving light flux RL from the light-receptive region SA0. As a result, the separation between the light receivable region LA0 and the slit image SL0 in FIG. 11B can be minimized. Thereby, contrast can be improved, and the occurrence of flare and/or ghost can be significantly reduced.

It should be noted that, in FIG. 12A and FIG. 12B, each of the illumination light flux IL and the light receiving light flux RL is illustrated as passing through the upper and lower iris. However, the configuration are not limited to this. For example, it is sufficient that the illumination light flux IL and the light receiving light flux RL are spatially separated in the iris. The illumination light flux IL may pass through the peripheral part, and the light receiving light flux RL may pass through the central part.

In some embodiments, at least one of the first ellipsoidal concave mirror 51 or the second ellipsoidal concave mirror 52 is a convex mirror whose reflective surface is formed in a convex shape (for example, ellipsoidal convex mirror). In some embodiments, at least one of the first ellipsoidal concave mirror 51 or the second ellipsoidal concave mirror 52 is a curved mirror whose reflective surface is a free-form surface.

In addition to the configuration shown in FIG. 1, the fundus imaging apparatus 1 may be provided with an alignment optical system for performing position matching between the eye E to be examined and the optical system.

Further, the fundus imaging apparatus 1 may have a configuration for providing a function associated with the inspection. For example, the fundus imaging apparatus 1 may be provided with a fixation optical system for projecting an optotype (fixation target) for fixating the eye E to be examined onto the fundus Ef of the eye E to be examined. Further, the fundus imaging apparatus 1 may be provided with any element or unit, such as a member for supporting the face of the examinee (chin rest, forehead pad, etc.).

In the present embodiment, the configuration according to the embodiments is configured to be capable of focusing on the fundus Ef of the eye E to be examined, using the movement of the slit 14 and/or the movement of the focusing lens 22. However, the configuration according to the embodiments is not limited to this. In some embodiments, the fundus imaging apparatus is configured to be capable of moving the entire optical system (illumination optical system 10, imaging optical system 20, hole mirror 30, and relay lens 40, or illumination optical system 10, imaging optical system 20, hole mirror 30, relay lens 40, first ellipsoidal concave mirror 51, and second ellipsoidal concave mirror 52) using a movement mechanism not shown.

The hole mirror 30 is an example of the "optical path dividing member" according to the embodiments. The first focal point F3 of the second ellipsoidal concave mirror 52 is an example of the "third focal point of the second ellipsoidal concave mirror" according to the embodiments. The second focal point F4 of the second ellipsoidal concave mirror 52 is an example of the "fourth focal point of the second ellipsoidal concave mirror" according to the embodiments.

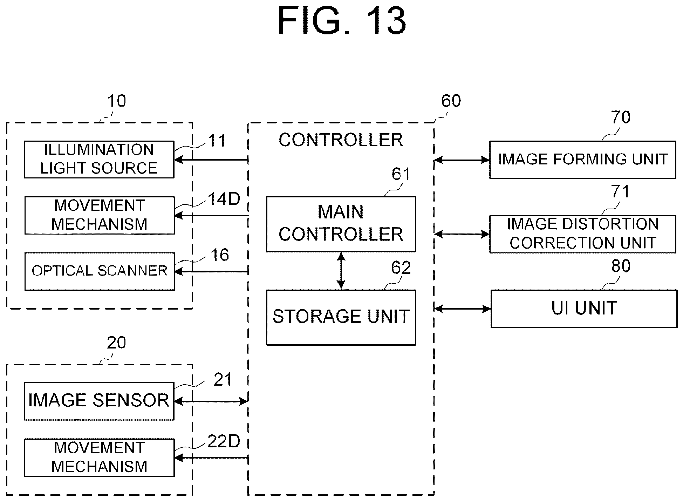

FIG. 13 shows an example of a configuration of a processing system of the fundus imaging apparatus 1 according to the embodiments. In FIG. 13, like reference numerals designate like parts as in FIGs. 1 to 3. The same description may not be repeated below.

The processing system of the fundus imaging apparatus 1 is configured with a controller 60 as a center. The controller 60 controls each part of the fundus imaging apparatus 1.

The controller 60 includes a main controller 61 and a storage unit 62. The functions of the main controller 61 are realized by a processor, for example. The storage unit 62 stores, in advance, computer programs for controlling the fundus imaging apparatus 1. Examples of the computer programs include an illumination light source control program, an image sensor control program, an optical scanner control program, an image forming program, an image distortion correcting program, and a program for user interface. The main controller 61 operates according to such the computer programs, and thereby the controller 60 performs the control processing.

(Main controller 61)

The main controller 61 controls each of the illumination optical system 10, the imaging optical system 20, an image forming unit 70, an image distortion correction unit 71, and a user interface (UI) unit 80.

Examples of the control for the illumination optical system 10 include control for the illumination light source 11, control for a movement mechanism 14D, and control for the optical scanner 16.

Examples of the control for the illumination light source 11 include turning the light source on and off, adjustment of the light quantity, and adjustment of an aperture.

The movement mechanism 14D moves the slit 14 in the optical axis direction of the illumination optical system 10. The main controller 61 outputs control signal(s) to the movement mechanism 14D to move the slit 14 in a direction corresponding to the control signal(s) by the amount of movement corresponding to the control signal(s). For example, the fundus imaging apparatus 1 is provided with an actuator that generates a driving force for moving the movement mechanism 14D and a transmission mechanism that transmits the driving force from the actuator to the movement mechanism 14D. The actuator includes a pulse motor, for example. The transmission mechanism includes a combination of gears, a rack and pinion, and the like, for example. The movement mechanism 14D receives the driving force generated by the actuator controlled by the main controller 61 from the transmission mechanism, and moves the slit 14 in the optical axis direction.

The control for the optical scanner 16 includes control of the angle of the deflection surface deflecting the illumination light. By controlling the angle of the deflection surface, the deflection direction (scan direction) of the illumination light can be controlled. By controlling an angular range of the deflection surface, the scan range (scan start position and scan end position) can be controlled. By controlling the speed of changing the angle of deflection surface, the scan speed can be controlled.

In some embodiments, in case that the orientation of the common plane of focal points is changeable, the optical scanner 16 is configured to be capable of changing the deflection direction of the illumination light in conjunction with the change in the deflected orientation of the common plane of focal points.

Examples of the control for the imaging optical system 20 include control for the image sensor 21, and control for a movement mechanism 22D.

The control for the image sensor 21 includes control of setting the light receivable region on the light receiving surface, and control for reading out the light receiving result(s) using the rolling shutter system (for example, setting of light receiving size corresponding to the size of the illumination pattern, or the like). Further, the control for the image sensor 21 includes the reset control, the exposure control, the charge transfer control, and the output control.

The movement mechanism 22D moves the focusing lens 22 in the optical axis direction of the imaging optical system 20. The main controller 61 outputs control signal(s) to the movement mechanism 22D to move the focusing lens 22 in a direction corresponding to the control signal(s) by the amount of movement corresponding to the control signal(s). For example, the fundus imaging apparatus 1 is provided with an actuator that generates a driving force for moving the movement mechanism 22D and a transmission mechanism that transmits the driving force from the actuator to the movement mechanism 22D. The actuator includes a pulse motor, for example. The transmission mechanism includes a combination of gears, a rack and pinion, and the like, for example. The movement mechanism 22D receives the driving force generated by the actuator controlled by the main controller 61 from the transmission mechanism, and moves the focusing lens 22 in the optical axis direction.

Examples of the control for the image forming unit 70 include image forming control for forming images of the eye E to be examined from the light receiving result(s) obtained using the image sensor 21.

Examples of the control for the image distortion correction unit 71 include image distortion correction control for correcting the distortion of the image formed by the image forming unit 70.

In some embodiments, the storage unit 62 stores, in advance, distortion correction parameter(s) for correcting the distortion of the pre-acquired image. The main controller 61 controls the image distortion correction unit 71 based on the distortion correction parameter(s) stored in the storage unit 62 to correct the distortion of the image formed by the image forming unit 70.

In some embodiments, the main controller 61 controls the image distortion correction unit 71 to identify the distortion of the image formed by the image forming unit 70 and to calculate the distortion correction parameter(s) based on the identified the distortion of the image. The calculated distortion correction parameter(s) is/are stored in the storage unit 62. The main controller 61 controls the image distortion correction unit 71 based on the calculated distortion correction parameter(s) to correct the distortion of the image formed by the image forming unit 70.

Examples of the control for the UI unit 80 include control for a display device and control for an operation device (input device).

(Storage unit 62)

The storage unit 62 stores various types of data. Examples of the data stored in the storage unit 62 include light receiving result(s) obtained by the image sensor 21, image data of an image formed by the image forming unit 70, the distortion correction parameter(s) used for the image distortion correction control performed by the image distortion correction unit 71, and information on eye to be examined. The information on the eye to be examined includes information on the examinee such as patient ID and name, and information on the eye to be examined such as identification information of the left/right eye.

The storage unit 62 further stores various types of programs and data to run the fundus imaging apparatus 1.

(Image forming unit 70)

The image forming unit 70 can form the light receiving image (fundus image) corresponding to the arbitrary light receivable region, based on the light receiving result(s) read out from the image sensor 21 using the rolling shutter system. The image forming unit 70 can sequentially form light receiving light images corresponding to the light receivable regions and form an image of the eye E to be examined from a plurality of the formed light receiving images. The various images (the various image data) formed by the image forming unit 70 are stored in the storage unit 62, for example.

For example, the image forming unit 70 includes a processor, and executes the function described above by performing processing corresponding to the program(s) stored in the storage unit or the like.

(Image distortion correction unit 71)

The image distortion correction unit 71 corrects the distortion of the image formed by the image forming unit 70. The image distortion correction unit 71 corrects the distortion of the image based on the predetermined distortion correction parameter(s), or the distortion correction parameter(s) corresponding to a degree of distortion of the image.

For example, the image distortion correction unit 71 performs distortion correction on the image by correcting pixel positions within each region so that one or more regions having polygonal shapes within an original image become regions having predetermined shapes. In some embodiments, the image distortion correction unit 71 performs distortion correction of the image solely in predetermined correction regions.

In some embodiments, the storage unit 62 stores the distortion correction parameters in advance for each predetermined divided region. The image distortion correction unit 71 performs distortion correction of the image based on distortion correction parameters corresponding to the divided region. Here, the distortion correction parameters have been read out from the storage unit 62 for each divided region of the image.

In some embodiments, the image distortion correction unit 71 further complementary performs image distortion correction on the image obtained by projecting the slit image onto the fundus Ef based on the shape of the opening formed in the slit 14 shown in FIG. 10A.

(UI unit 80)

The UI unit 80 has a function for exchanging information between a user and the fundus imaging apparatus 1. The UI unit 80 includes the display device and the operation device. The display device may include a display unit, and it may include another display device. The display device displays various information. The display device includes a liquid crystal display, for example. The display device displays the above information under the control of the main controller 61. Examples of the information displayed on the display device include information corresponding to the control result by the controller 60, information (image) corresponding to the calculation result by the image forming unit 70. The operation device includes various hardware keys and/or various software keys. Upon receiving the operation content for the operation device, the main controller 61 can output a control signal corresponding to the operation content to each part of the fundus imaging apparatus. At least a part of the display device and at least a part of the operation device may be configured integrally. One example of this is the touch panel display.

<Operation>

Next, the operation of the fundus imaging apparatus 1 according to the embodiments will be described.

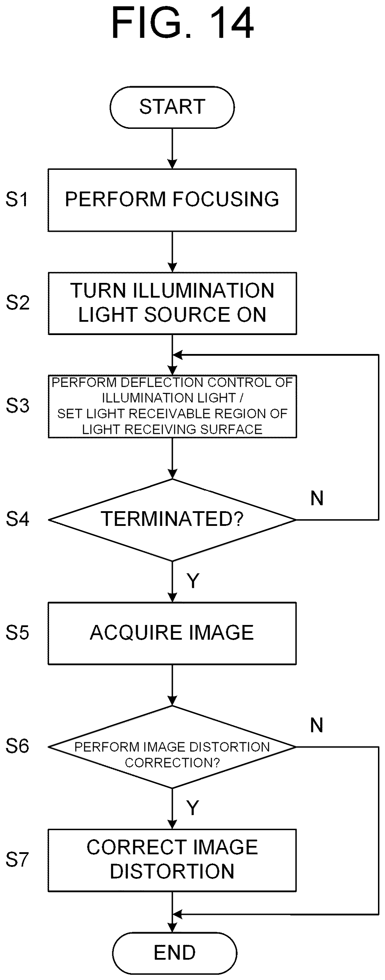

FIG. 14 shows an example of the operation of the fundus imaging apparatus 1 according to the embodiments. FIG. 14 represents a flowchart of the example of the operation of the fundus imaging apparatus 1 according to the embodiments. The storage unit 62 stores computer program(s) for realizing the processing shown in FIG. 14. The main controller 61 operates according to the computer program(s), and thereby the main controller 61 performs the processing shown in FIG. 14.

In FIG. 14, it is assumed that the eye E to be examined has been arranged at a predetermined eye to be examined position (the second focal point F4 of the second ellipsoidal concave mirror 52), and that the illumination range (scan range) of the illumination light on the fundus Ef has been determined in advance.

(S1: Perform focusing)

First, the main controller 61 controls at least one of the movement mechanism 14D or the movement mechanism 22D to adjust the focal position of the illumination optical system 10 and the focal position of the imaging optical system 20 with respect to the fundus Ef of the eye E to be examined.

(S2: Turn illumination light source on)

Next, the main controller 61 controls the illumination light source 11 to turn the illumination light source 11 on.

The light output from the illumination light source 11 passes through the opening(s) formed in the iris aperture 12, is transmitted through the relay lens 13, passes through the opening(s) formed in the slit 14, is transmitted through the relay lens 15, and is guided to the hole mirror 30 as the slit-shaped illumination light via the optical scanner 16 and the relay lens 17.

(S3: Perform deflection control of illumination light / Set light receivable region of light receiving surface)

Subsequently, the main controller 61 controls the optical scanner 16 to set the orientation of the deflection surface in a predetermined deflection direction in order to illuminate a predetermined illumination range (scan range), and starts deflection control of the illumination light that sequentially changes the orientation of the deflection surface within a predetermined deflection angular range. In other words, the main controller 61 starts scanning of the illumination light onto the fundus Ef.

In some embodiments, the main controller 61 controls the optical scanner 16 to perform deflection control of the illumination light in synchronization with the movement of the light receivable region that can be arbitrarily set on the image sensor 21.

In some embodiments, the main controller 61 controls the image sensor 21 to set the light receivable region of the returning light on the light receiving surface. Here, the light receivable region corresponds to the illumination region of the illumination light on the fundus Ef. For example, the illumination region on the fundus Ef can be identified based on the deflection angle of the deflection surface of the optical scanner 16. The main controller 61 can set the light receivable region on the light receiving surface of the image sensor 21, corresponding to the deflection direction of the deflection surface of the optical scanner 16 that is changed sequentially.

The illumination light that has been guided to the hole mirror 30 is reflected on the reflective surface on the peripheral part of the hole part, passes through the relay lens 40, is focused on the first focal point F1 of the first ellipsoidal concave mirror 51, is guided to the reflective surface of the first ellipsoidal concave mirror 51, and is reflected on this reflective surface. The illumination light reflected on the reflective surface of the first ellipsoidal concave mirror 51 is guided to the reflective surface of the second ellipsoidal concave mirror 52 via the second focal point F2 (first focal point F3) of the first ellipsoidal concave mirror 51. The illumination light guided to the reflective surface of the second ellipsoidal concave mirror 52 is reflected on this reflective surface, enters the eye of the eye E to be examined arranged at the second focal point F4 of the second ellipsoidal concave mirror 52, and is irradiated onto the fundus Ef.

The returning light from the eye E to be examined, which includes the returning light from the fundus Ef, travels in the same path as the outward path in the opposite direction, passes through the hole part formed in the hole mirror 30, passes through the focusing lens 22, and is received on the light receiving surface of the image sensor 21. On the light receiving surface of the image sensor 21, the light receivable region of the returning light corresponding to the illumination region of the illumination on the fundus Ef is set. Thereby, the returning light alone from fundus Ef can be received while suppressing the effect of unnecessary scattered light.

(S4: Terminated?)

Subsequently, the main controller 61 determines whether or not to terminate the scanning of the illumination light for the fundus Ef. For example, the main controller 61 can determine whether or not to terminate the scanning of the illumination light for the fundus Ef, by determining whether or not the deflection angle of the deflection surface of the hole mirror 30, which is changed sequentially, is within the predetermined deflection angular range.

When it is determined to terminate the scanning of the illumination light for the fundus Ef (S4: Y), the operation of the fundus imaging apparatus 1 proceeds to step S5. When it is determined not to terminate the scanning of the illumination light for the fundus Ef (S4: N), the operation of the fundus imaging apparatus 1 proceeds to step S3.

(S5: Acquire image)

When it is determined in step S4 to terminate the scanning of the illumination light for the fundus Ef (S4: Y), the main controller 61 controls the image forming unit 70 to form an image of the eye E to be examined based on the light receiving result(s) read out from the image sensor 21. In some embodiments, the image forming unit 70 sequentially forms the light receiving images based on the light receiving results read from the image sensor 21 in step S3, and forms an image of the eye E to be examined from the plurality of formed light receiving images.

(S6: Perform image distortion correction?)

Next, the main controller 61 determines whether or not to perform image distortion correction on the image acquired in step S5.

In some embodiments, it is predetermined whether or not to perform image distortion correction before executing the flow shown in FIG. 14. In some embodiments, the user operates the UI unit 80. And, the main controller 61 determines whether or not to perform image distortion correction based on the operation content for the UI unit 80 by the user.

When it is determined to perform image distortion correction (S6: Y), the operation of the fundus imaging apparatus 1 proceeds to step S7. When it is determined not to perform image distortion correction (S6: N), the fundus imaging apparatus 1 terminates the operation (END).

(S7: Correct image distortion)

When it is determined in step S6 to perform image distortion correction (S6: Y), the main controller 61 controls the image distortion correction unit 71 to perform image distortion correction on the image acquired in step S5.

The image distortion correction unit 71 complementary perform image distortion correction on the image acquired in step S5, as described above.

Subsequent to step S7, or when it is determined in step S6 not to perform image distortion correction (S6: N), the fundus imaging apparatus 1 terminates the operation (END).

As described above, according to the embodiments, the slit 14 is arranged so that the common plane of focal points including the four focal points of the two ellipsoidal concave mirrors is approximately parallel to the longitudinal direction of the slit image projected onto the fundus Ef, and the fundus Ef is scanned so that the slit image is moved in the direction orthogonal to the longitudinal direction of the slit image. Thereby, the contrast can be improved and the occurrence of the flare and/or ghost can be suppressed while securing a wide imaging angle of view at low cost with a simple configuration, without the need for additional correction optical systems. In addition, advanced optical design is not required.

Further, at least one of the both ends of the opening, which is formed in the slit 14, in the longitudinal direction is displaced in the shorter direction of the opening with reference to the longitudinal direction passing through a center of the opening. Thereby, the contrast degradation caused by distortion of the slit image can be suppressed with a simple configuration, without adding optical elements.

Furthermore, by making the scan length variable, the scan range of the illumination light can be set as desired.

<First modification example>

The configuration of the fundus imaging apparatus according to the embodiments is not limited to the configuration of the fundus imaging apparatus 1. For example, the fundus imaging apparatus 1 according to the embodiments may further be provided with an OCT optical system.

Hereinafter, in the embodiments, a case of using the swept source type OCT method in the measurement or the imaging (shooting) using OCT will be described. However, the configuration according to the embodiments can also be applied to a fundus imaging apparatus using other type of OCT (for example, spectral domain type OCT).

In the following, the fundus imaging apparatus according to a first modification example of the embodiments will be described focusing on differences from the fundus imaging apparatus 1 according to the embodiments.

<Configuration>

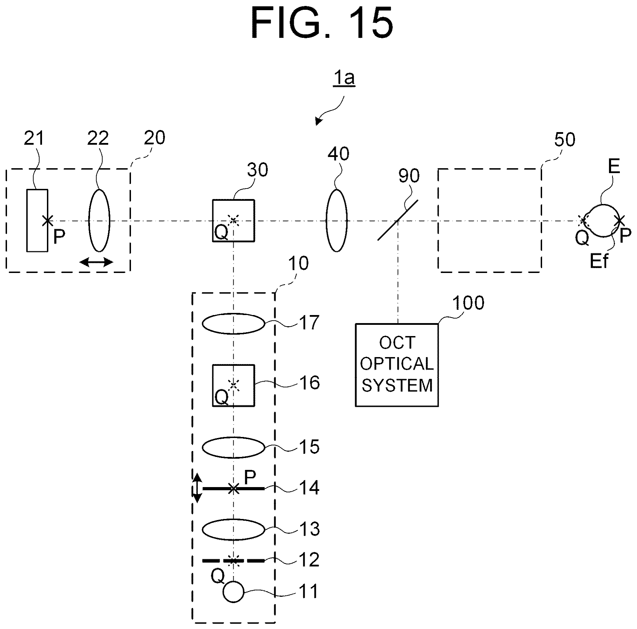

FIG. 15 shows an example of a configuration of an optical system of a fundus imaging apparatus according to the first modification example of the embodiments. In FIG. 15, like reference numerals designate like parts as in FIG. 1, and the redundant explanation may be omitted as appropriate.

The difference between a configuration of an optical system of a fundus imaging apparatus 1a according to the first modification example of the embodiments and the configuration of the optical system of the fundus imaging apparatus 1 according to the embodiments is that an OCT optical system 100 and a dichroic mirror 90 are added to the configuration of the optical system of the fundus imaging apparatus 1. The dichroic mirror 90 is arranged between the relay lens 40 and the relay optical system 50, as an optical path coupling separating member (optical path dividing member). In some embodiments, the dichroic mirror 90 is arranged between the hole mirror 30 and the relay lens 40.

The dichroic mirror 90 is an optical path coupling separating member that spatially separates the optical path of the illumination light and an optical path of the OCT optical system 100 (or that couples the optical path of the illumination light and the optical path of the OCT optical system 100). The dichroic mirror 90 reflects measurement light from the OCT optical system 100 to guide the measurement light to the relay lens 40, and reflects returning light of the measurement light from eye E to be examined to guide the returning light to the OCT optical system 100. Further, the dichroic mirror 90 transmits the illumination light that has been guided via the relay lens 40, and transmits the returning light from the eye E to be examined to guide the returning light to the relay lens 40.

(OCT optical system 100)

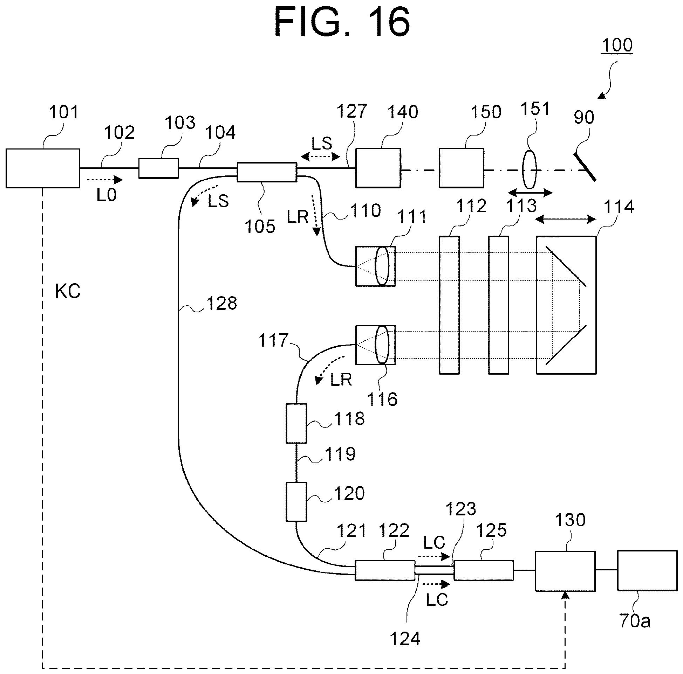

FIG. 16 shows an example of the configuration of the OCT optical system 100 in FIG. 13. In FIG. 16, parts similar to those in FIG. 15 are denoted by the same reference symbols, and description thereof is omitted as appropriate.

The OCT optical system 100 is provided with an optical system for performing OCT measurement (or OCT imaging) on the eye E to be examined. This optical system is an interference optical system that splits light from a wavelength sweeping type (i.e., a wavelength scanning type) light source into the measurement light and reference light, makes the measurement light returning from the eye E to be examined and the reference light having traveled through the reference optical path interfere with each other to generate interference light, and detects the interference light. The detection result (detection signal) of the interference light obtained by the interference optical system is a signal indicating a spectrum of the interference light, and is sent to an image forming unit 70a, a data processor 75a, and the like which are described below.

Like swept source type fundus imaging apparatuses commonly used, the OCT light source 101 includes a wavelength sweeping type (i.e., a wavelength scanning type) light source capable of sweeping (scanning) the wavelengths of emitted light. A laser light source including a resonator and emitting light having a center wavelength of 1050 nm, for example, is used as the wavelength sweeping type light source. The OCT light source 101 temporally changes the output wavelength in the near infrared wavelength band which cannot be visually recognized by the human eye.

Light L0 output from the OCT light source 101 is guided to the polarization controller 103 through the optical fiber 102, and the polarized wave state of the light L0 is adjusted. The polarization controller 103, for example, applies external stress to the looped optical fiber 102 to thereby adjust the polarization state of the light L0 guided through the optical fiber 102.

The light L0 whose polarization state has been adjusted by the polarization controller 103 is guided to the fiber coupler 105 through the optical fiber 104, and is split into the measurement light LS and the reference light LR.

The reference light LR is guided to the collimator 111 through the optical fiber 110. The reference light LR is converted into a parallel light flux by the collimator 111. Then, the reference light LR is guided to the optical path length changing unit 114 via an optical path length correction member 112 and a dispersion compensation member 113. The optical path length correction member 112 acts so as to match the optical path length of the reference light LR with the optical path length of the measurement light LS. The dispersion compensation member 113 acts so as to match the dispersion characteristics between the reference light LR and the measurement light LS.

The optical path length changing unit 114 is movable in directions indicated by the arrow in FIG. 16, thereby changing the optical path length of the reference light LR. Through such movement, the optical path length of the reference light LR is changed. The change in the optical path length is used for the correction of the optical path length according to the axial length of the eye E to be examined, for the adjustment of the interference state, or the like. The optical path length changing unit 114 includes, for example, a corner cube and a movement mechanism for moving the corner cube. In this case, the corner cube in the optical path length changing unit 114 changes the traveling direction of the reference light LR that has been made into the parallel light flux by the collimator 111 in the opposite direction. The optical path of the reference light LR incident on the corner cube and the optical path of the reference light LR emitted from the corner cube are parallel.

The reference light LR that has traveled through the optical path length changing unit 114 passes through the dispersion compensation member 113 and the optical path length correction member 112, is converted from the parallel light flux to the convergent light flux by a collimator 116, and enters an optical fiber 117. The reference light LR that has entered the optical fiber 117 is guided to a polarization controller 118, and the polarization state of the reference light LR is adjusted. Then the reference light LR is guided to an attenuator 120 through an optical fiber 119, and the light amount of the reference light LR is adjusted. After that, the reference light LR is guided to a fiber coupler 122 through an optical fiber 121.

Meanwhile, the measurement light LS generated by the fiber coupler 105 is guided through the optical fiber 127, and is made into the parallel light flux by the collimator lens unit 140. The measurement light LS which has been made into the parallel light flux is deflected one-dimensionally or two-dimensionally using an optical scanner (optical scanner for OCT) 150.

The collimator lens unit 140 includes a collimator lens. The collimator lens is disposed on an optical axis of the interference optical system included in the OCT optical system 100. The collimator lens converts a light flux of the measurement light emitted from the end of an optical fiber into a parallel light flux. Here, the optical fiber is connected to the OCT optical system 100 and guides the measurement light LS to the end. The end of the optical fiber is arranged at the fundus conjugate position P, for example.

The optical scanner 150 (deflection surface) can be arranged at the iris conjugate position Q. In case of deflecting the illumination light in a one-dimensionally manner, the optical scanner 150 includes a galvanometer scanner that deflects the measurement light LS within a predetermined deflection angular range with reference to a predetermined deflection direction. In case of deflecting the illumination light in a two-dimensionally manner, the optical scanner 150 includes a first galvanometer scanner and a second galvanometer scanner. The first galvanometer scanner deflects the measurement light LS so as to move the irradiated position in a horizontal direction (for example, X direction) orthogonal to an optical axis of the OCT optical system 100. The second galvanometer scanner deflects the measurement light LS deflected by the first galvanometer scanner so as to move the irradiated position in a vertical direction (for example, Y direction) orthogonal to the optical axis of the OCT optical system 100. Examples of scan mode for moving the irradiated position of the measurement light LS using the optical scanner 150 include a horizontal scan, a vertical scan, a cross scan, a radial scan, a circle scan, a concentric scan, a helical (spiral) scan, and a Lissajous scan.

The measurement light LS deflected by the optical scanner 150 passes through a focusing lens 151, is reflected by the dichroic mirror 90 to guide to the reflective surface of the first ellipsoidal concave mirror 51, and is guided to the eye E to be examined along the same path as the illumination light from the illumination optical system 10. The focusing lens 151 is movable along the optical path of the measurement light LS (optical axis of the OCT optical system 100). The focusing lens 151 is moved along the optical path of the measurement light LS using a movement mechanism (not shown), under the control from the controller (controller 60a) described below.

The measurement light LS reflected on the reflective surface of the second ellipsoidal concave mirror 52 enters the eye E to be examined at the second focal point F4 (eye to be examined position) through the pupil. The measurement light LS is scattered (and reflected) at various depth positions of the eye E to be examined. The returning light of the measurement light LS including such backscattered light advances through the same path as the outward path in the opposite direction and is guided to the fiber coupler 105, and then reaches the fiber coupler 122 through the optical fiber 128.

The fiber coupler 122 combines (interferes) the measurement light LS incident through the optical fiber 128 and the reference light LR incident through the optical fiber 121 to generate interference light. The fiber coupler 122 generates a pair of interference light LC by splitting the interference light generated from the measurement light LS and the reference light LR at a predetermined splitting ratio (for example, 1 : 1). The pair of the interference light LC emitted from the fiber coupler 122 is guided to the detector 125 through the optical fibers 123 and 124, respectively.

The detector 125 is, for example, a balanced photodiode that includes a pair of photodetectors for respectively detecting the pair of interference light LC and outputs the difference between the pair of detection results obtained by the pair of photodetectors. The detector 125 sends the detection result(s) (i.e., interference signal(s)) to the DAQ (Data Acquisition System) 130. A clock KC is supplied from the OCT light source 101 to the DAQ 130. The clock KC is generated in the OCT light source 101 in synchronization with the output timing of each wavelength sweeping (scanning) within a predetermined wavelength range performed by the wavelength sweeping type light source. For example, the OCT light source 101 optically delays one of the two pieces of branched light obtained by branching the light L0 of each output wavelength, and then generates the clock KC based on the result of the detection of the combined light of the two pieces of branched light. The DAQ 130 performs sampling of the detection result obtained by the detector 125 based on the clock KC. The DAQ 130 sends the sampled detection result(s) obtained by the detector 125 to the image forming unit 70a, the data processor 75a, and the like. The image forming unit 70a (or data processor 75a) applies Fourier transform and the like to the spectral distribution based on the detection result(s) obtained by the detector 125, for example, with respect to a series of wavelength scans (for each A-line) to form the reflection intensity profile in each A-line. In addition, the image forming unit 70a forms image data by applying imaging processing to the reflection intensity profiles of the each A-line.

It should be noted that the difference between the optical path length of the measurement light and the optical path length of the reference light is changed by changing the optical path length of the reference light, in FIG. 16. However, the configuration according to the embodiments is not limited to this. For example, by changing the optical path length of the measurement light, the difference between the optical path length of the measurement light and the optical path length of the reference light may be configured to be changed.

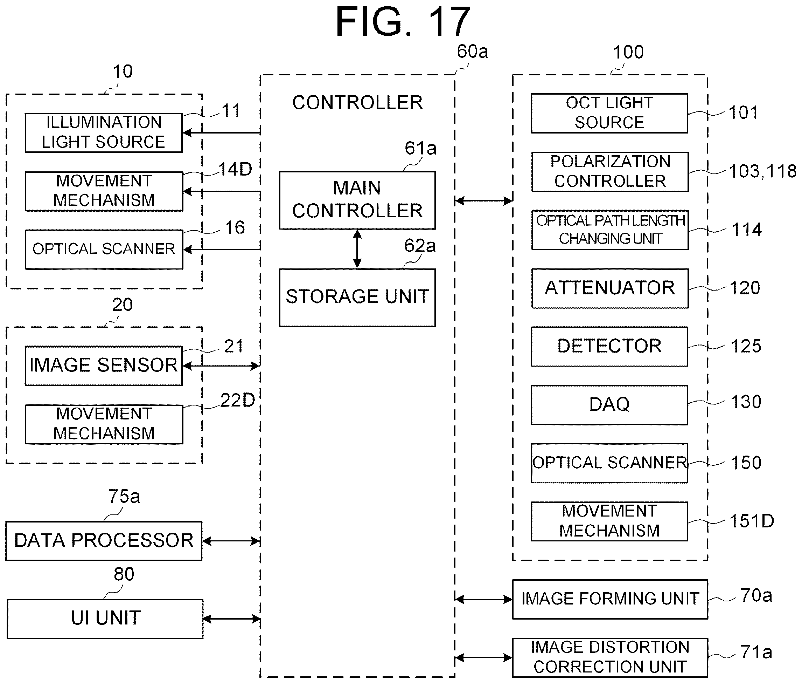

FIG. 17 shows an example of a configuration of a processing system of the fundus imaging apparatus 1a according to the first modification example of the embodiments. In FIG. 17, like reference numerals designate like parts as in FIG. 13, FIG. 15, or FIG. 16. The same description may not be repeated.

The differences between the configuration of the processing system of the fundus imaging apparatus 1a according to the first modification example and the configuration of the processing system of the fundus imaging apparatus 1 according to the embodiments are that the controller 60a is provided instead of the controller 60, that the image forming unit 70a is provided instead of the image forming unit 70, that the image distortion correction unit 71a is provided instead of the image distortion correction unit 71, and that the data processor 75a and the OCT optical system 100 are added.

The controller 60a includes a main controller 61a and a storage unit 62a, and in addition to the controls that can be performed by the controller 60, the controller 60a performs control for the image forming unit 70a, the image distortion correction unit 71a, the data processor 75a, and the OCT optical system 100. The functions of the main controller 61a are realized by a processor, for example, in the same way as the main controller 61. The storage unit 62a stores, in advance, computer programs for controlling the fundus imaging apparatus 1a, in the same way as the storage unit 62. Examples of the computer programs include an illumination light source control program, an image sensor control program, an optical scanner control program, an image forming program, an image distortion correction program, a data processing program, an OCT optical system control program, and a program for user interface. The main controller 61a operates according to the computer programs, and thereby the controller 60a performs the control processing.

The main controller 61a controls each of the illumination optical system 10, the imaging optical system 20, the image forming unit 70a, the image distortion correction unit 71a, the data processor 75a, the OCT optical system 100, and the UI unit 80.

Examples of the control for the OCT optical system 100 include control for the OCT light source 101, operation control for the polarization controllers 103 and 118, movement control for the optical path length changing unit 114, operation control for the attenuator 120, control for the detector 125, control for the DAQ 130, control for the optical scanner 150, and control for a movement mechanism 151D.

Examples of the control for the OCT light source 101 include turning the light source on and off, adjustment of the light quantity, adjustment of an aperture, and the like. Examples of the control for the detector 125 include adjustment of exposure of a detecting element, adjustment of gain of a detecting element, adjustment of detecting rate of a detecting element, and the like. Examples of the control for the optical scanner 150 include control of the scan direction, the scan position, the scan range, and scan speed by the optical scanner 150.

The movement mechanism 151D moves the focusing lens 151 in the optical axis direction of the OCT optical system 100. The main controller 61a outputs control signal(s) to the movement mechanism 151D to move the focusing lens 151 in a direction corresponding to the control signal(s) by the amount of movement corresponding to the control signal(s). For example, the fundus imaging apparatus 1 is provided with an actuator that generates a driving force for moving the movement mechanism 151D and a transmission mechanism that transmits the driving force from the actuator to the movement mechanism 151D. The actuator includes a pulse motor, for example. The transmission mechanism includes a combination of gears, a rack and pinion, and the like, for example. The movement mechanism 151D receives the driving force generated by the actuator controlled by the main controller 61a from the transmission mechanism, and moves the focusing lens 151 in the optical axis direction. Thereby, the focusing position of the measurement light LS is changed. The focusing position of the measurement light LS corresponds to the depth position (z position) of the beam waist of the measurement light LS.

Examples of the control for the image forming unit 70a include control of forming OCT images based on the detection result(s) of the interference light obtained by the OCT optical system 100, in addition to the image forming control that forms images of the eye E to be examined from the light receiving result(s) obtained using the image sensor 21.

Examples of the control for the image distortion correction unit 71a include image distortion correction control for correcting the distortion of the image formed by the image forming unit 70a.

Examples of the data processor 75a include control of image processing for the images formed by the image forming unit 70a and a control of analysis processing for images. The data processor 75a may include the image distortion correction unit 71a, and may be configured to realize the functions of the image distortion correction unit 71a.

The image forming unit 70a can form the light receiving image (fundus image) corresponding to the arbitrary light receivable region based on the light receiving result(s) read out from the image sensor 21, in the same way as the image forming unit 70. The image forming unit 70a can sequentially form light receiving light images corresponding to the light receivable regions and form an image of the eye E to be examined from a plurality of the formed light receiving images.

Further, the image forming unit 70a forms image data of the OCT image (tomographic image) based on the detection signals input from the DAQ 130 (detector 125) and pixel position signals input from the controller 60a. Examples of the OCT image formed by the image forming unit 70a include an A-scan image and a B-scan image. The B-scan image is formed by arranging the A-scan images in the B-scan direction. As with the conventional swept source OCT, the image forming processing includes noise removal (noise reduction), filtering, dispersion compensation, fast Fourier transform (FFT), and the like. In the case of employing an OCT apparatus of another type, the image forming unit 70a performs known processing according to the type employed. The various images (the various image data) formed by the image forming unit 70a are stored in the storage unit 62a, for example.

The image distortion correction unit 71a corrects the distortion of the image formed by the image forming unit 70a, in the same manner as the image distortion correction unit 71.

In some embodiments, the image distortion correction unit 71a corrects the distortion of the image formed by the image forming unit 70a. In this case, the storage unit 62a stores, in advance, distortion correction parameter(s) for correcting the distortion of the pre-acquired image. The image distortion correction unit 71a can correct the distortion of the image formed based on the image forming unit 70a, based on the distortion correction parameter(s) stored in the storage unit 62a.

The data processor 75a processes images formed based on the light receiving result(s) obtained by the imaging optical system 20 or data acquired by performing OCT measurement on the eye E to be examined. The data processor 75a can perform various kinds of image processing and various kinds of analysis processing on the image formed by the image forming unit 70a or the image on which the distortion correction has been performed by the image distortion correction unit 71a. For example, the data processor 75a performs various types of image correction processing such as brightness correction.

The data processor 75a performs known image processing such as interpolation processing for interpolating pixels between the OCT images to form image data of the three-dimensional image of the fundus Ef. It should be noted that the image data of the three-dimensional image means image data in which the positions of pixels are defined in a three-dimensional coordinate system. Examples of the image data of the three-dimensional image include image data defined by voxels three-dimensionally arranged. Such image data is referred to as volume data or voxel data. In case of displaying an image based on volume data, the data processor 75a performs image rendering processing (e.g., volume rendering, maximum intensity projection (MIP)) on the volume data to form image data of a pseudo three-dimensional image taken from a specific view direction. The pseudo three-dimensional image is displayed on a display device included in the UI unit 80.

Further, the three-dimensional image data may be stack data of a plurality of tomographic images. The stack data is image data formed by three-dimensionally arranging tomographic images along a plurality of scan lines based on positional relationship of the scan lines. That is, the stack data is image data obtained by representing tomographic images, which are originally defined in their respective two-dimensional coordinate systems, by a single three-dimensional coordinate system. That is, the stack data is image data formed by embedding tomographic images into a single three-dimensional space.

The data processor 75a can form a B-mode image (longitudinal cross-sectional image, axial cross-sectional image) in an arbitrary cross section, a C-mode image (transverse section image, horizontal cross-sectional image) in an arbitrary cross section, a projection image, a shadowgram, etc., by performing various renderings on the acquired three-dimensional data set (volume data, stack data, etc.). An image in an arbitrary cross section such as a B-mode image or a C-mode image is formed by selecting pixels (voxels) on a designated cross section from the three-dimensional data set. The projection image is formed by projecting the three-dimensional data set in a predetermined direction (Z direction, depth direction, axial direction). The shadowgram is formed by projecting a part of the three-dimensional data set (for example, partial data corresponding to a specific layer) in a predetermined direction. An image having a viewpoint on the front side of the eye to be examined, such as the C-mode image, the projection image, and the shadowgram, is called a front image (en-face image).

The data processor 75a can build (form) the B-mode image or the front image (blood vessel emphasized image, angiogram) in which retinal blood vessels and choroidal blood vessels are emphasized (highlighted), based on data (for example, B-scan image data) acquired in time series by OCT. For example, the OCT data in time series can be acquired by repeatedly scanning substantially the same site of the eye E to be examined.

In some embodiments, the data processor 75a compares the B-scan images in time series acquired by performing B-scan on substantially the same site, converts the pixel value of a change portion of the signal intensity into a pixel value corresponding to the change portion, and builds the emphasized image in which the change portion is emphasized. Further, the data processor 75a forms an OCTA image by extracting information of a predetermined thickness at a desired site from a plurality of built emphasized images and building as an en-face image.

An image (for example, a three-dimensional image, a B-mode image, a C-mode image, a projection image, a shadowgram, and an OCTA image) generated by the data processor 75a is also included in the OCT image.

Further, the data processor 75a performs predetermined analysis processing on the image formed based on the light receiving result(s) obtained by the imaging optical system 20, the detection result(s) of the interference light acquired by the OCT measurement, or the OCT image formed based on the detection result(s). Examples of the predetermined analysis processing include identification of a predetermined site (tissue, lesion) of the eye E to be examined; calculation of a distance, area, angle, ratio, or density between designated sites (distance between layers, interlayer distance); calculation by a designated formula; identifying of the shape of a predetermined site; calculation of these statistics; calculation of distribution of the measured value or the statistics; image processing based on these analysis processing results, and the like. Examples of the predetermined tissue include a blood vessel, an optic disc, a fovea, a macula, and the like. Examples of the predetermined lesion include a leukoma, a hemorrhage, and the like.

The fundus imaging apparatus 1a may include a movement mechanism that moves the OCT optical system 100 in a one-dimensional direction or two-dimensional directions, which intersect(s) the optical axis of the OCT optical system 100. In this case, the main controller 61a controls this movement mechanism to move the OCT optical system 100 relative to the dichroic mirror 90 in the one-dimensional direction or two-dimensional directions, which intersect(s) the optical axis of the OCT optical system 100. This allows to move the scan range using the optical scanner 150 in the OCT optical system 100 and to scan the wide-angle scan range on the fundus Ef.

The dichroic mirror 90 is an example of the "optical path coupling separating member" according to the embodiments.

<Operation>

Next, the operation of the fundus imaging apparatus 1a according to the first modification example of the embodiments will be described.

The fundus imaging apparatus 1a can perform OCT measurement using the OCT optical system 100 in parallel with the scan control for the fundus Ef using the slit-shaped illumination light from the illumination optical system 10. In some embodiments, the fundus imaging apparatus 1a performs OCT measurement using the OCT optical system 100 in a state where the scan control for the fundus Ef using the slit-shaped illumination light from the illumination optical system 10 is stopped. Hereafter, the control of OCT measurement that can be performed in parallel with the scan control for the fundus Ef using the slit-shaped illumination light will be described.

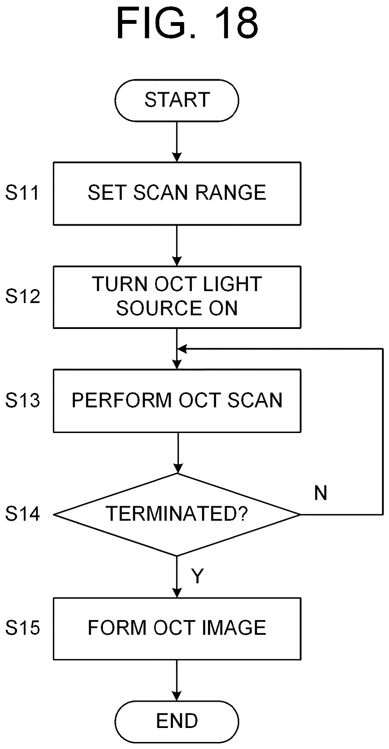

FIG. 18 shows an example of an operation of the fundus imaging apparatus 1a according to the first modification example of the embodiments. FIG. 18 represents a flowchart of the example of the operation of the fundus imaging apparatus 1a according to the first modification example. The storage unit 62a stores computer program(s) for realizing the processing shown in FIG. 18. The main controller 61a operates according to the computer programs, and thereby the main controller 61a performs the processing shown in FIG. 18.

It is assumed that the eye E to be examined is arranged at a predetermined eye to be examined position (the second focal point F4 of the second ellipsoidal concave mirror 52), in FIG. 18.

(S11: Set scan range)

First, the main controller 61a sets the scan range of the optical scanner 150. The main controller 61a can set the scan start position, the scan end position, the scan speed (scan frequency), etc. by the optical scanner 150, along with the scan range.

In some embodiments, the user can designate the scan mode or the operation mode by operating the operation device in the UI unit 80. When the scan mode (for example, horizontal scan, vertical scan) is designated by operating the operation device by the user, the main controller 61a analyzes operation information from the operation device to identify the designated scan mode. When the operation mode is designated by operating the operation device by the user, the main controller 61a analyzes the operation information to identify a scan mode (for example, horizontal scan, vertical scan) designated in advance in the designated operation mode (for example, OCT measurement mode).

(S12: Turn OCT light source on)

Subsequently, the main controller 61a controls the OCT light source 101 to turn the OCT light source 101 on. In some embodiments, the main controller 61a performs step S12 in synchronization with the turning on control of the illumination light source 11 in step S2 shown in FIG. 14.

In some embodiments, the main controller 61a performs controls of adjusting focusing and of adjusting polarization. For example, the main controller 61a controls the OCT optical system 100 to perform OCT measurement, after controlling the movement mechanism 151D to move the focusing lens by a predetermined distance. The main controller 61a controls the data processor 75a to determine the focus state of the measurement light LS based on the detection result of the interference light acquired by the OCT measurement. For example, the data processor 75a analyzes the detection results of the interference light acquired by the OCT measurements to calculate predetermined evaluation value(s) relating to the image quality of OCT images and to determine the focus state based on the calculated evaluation value(s). When it is determined that the focus state is not appropriate based on the determination result of the data processor 75a, the main controller 61a controls a movement mechanism 151D again and repeats this until it is determined that the focus state of the measurement light LS is appropriate.

Further, for example, the main controller 61a controls the OCT optical system 100 to perform OCT measurement after controlling at least one of the polarization controller 103 or the polarization controller 118 to change the polarization state of at least one of the light L0 or the measurement light LS by a predetermined amount. And then, the main controller 61a controls the image forming unit 70a to form the OCT image on the basis of the acquired detection result of the interference light. The main controller 61a controls the data processor 75a to determine the image quality of the OCT image acquired by performing the OCT measurement. When it is determined that the polarization state of the measurement light LS is not appropriate based on the determination result of the data processor 75a, the main controller 61a controls the polarization controllers 103 and 118 again and repeats this until it is determined that the polarization state of the measurement light LS is appropriate.

(S13: Perform OCT scan)

Subsequently, the main controller 61a controls the optical scanner 150 to deflect the measurement light LS generated based on the light L0 emitted from the OCT light source 101 to scan a predetermined site on the fundus Ef of the eye E to be examined with the deflected measurement light LS. The detection result of the interference light acquired by the OCT measurement is sampled by the DAQ 130 and is stored as the interference signal in the storage unit 62a or the like.

(S14: Terminated?)

Subsequently, the main controller 61a determines whether or not to terminate the OCT scan for the fundus Ef. For example, the main controller 61a can determine whether or not to terminate the OCT scan for the fundus Ef, by determining whether or not the deflection angle of the deflection surface of the optical scanner 150, which is changed sequentially, is within the predetermined deflection angular range.

When it is determined to terminate the OCT scan for the fundus Ef (S14: Y), the operation of the fundus imaging apparatus 1a proceeds to step S15. When it is determined not to terminate the OCT scan for the fundus Ef (S14: N), the operation of the fundus imaging apparatus 1a proceeds to step S13.

(S15: Form OCT image)

When it is determined in step S14 to terminate the OCT scan for the fundus Ef (S14: Y), the main controller 61a controls the image forming unit 70a to form a plurality of A-scan images of the fundus Ef along the B-scan direction, based on the interference signal(s) acquired by performing OCT scan in step S13. In some embodiments, the main controller 61a controls the data processor 75a to form OCT images such as three-dimensional OCT images, B-mode images, C-mode images, projection images, shadowgrams, and OCTA images.

This terminates the operation of the fundus imaging apparatus 1a (END).

As described above, according to the first modification example of the embodiments, in addition to the effects obtained in the embodiments, OCT measurement (OCT imaging) can be performed on any position on the fundus being observed at a large wide angle, without sharing an optical scanner for OCT scanning and an optical scanner for deflection of illumination light.

<Second modification example>

The configuration of the fundus imaging apparatus according to the embodiments is not limited to the configuration according to the embodiments or the first modification example thereof described above. For example, in the configuration shown in FIG. 2 and FIG. 3, three or more curved mirrors (aspheric mirrors) may be provided instead of the first ellipsoidal concave mirror 51 and the second ellipsoidal concave mirror 52.

In a second modification example, two concave mirrors and a single convex mirror are provided instead of the first ellipsoidal concave mirror 51 and the second ellipsoidal concave mirror 52.

In the following, the fundus imaging apparatus according to the second modification example of the embodiments will be described focusing on differences from the fundus imaging apparatus 1 according to the embodiments.

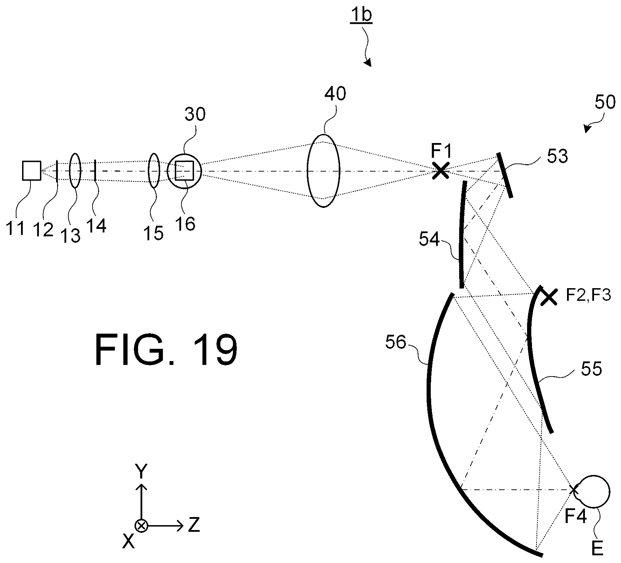

FIG. 19 shows an example of a configuration of an optical system of a fundus imaging apparatus 1b according to the second modification example of the embodiments. In FIG. 19, like reference numerals designate like parts as in FIG. 3, and the redundant explanation may be omitted as appropriate.

The difference between the configuration of the fundus imaging apparatus 1b according to the second modification example and the configuration of the fundus imaging apparatus 1 according to the embodiments is that a reflective mirror 53 as a plane mirror, a hyperboloidal concave mirror 54 as a concave mirror, a hyperboloidal convex mirror 55 as a convex mirror, and an ellipsoidal concave mirror 56 as a concave mirror are provided instead of the first ellipsoidal concave mirror 51 and the second ellipsoidal concave mirror 52.

The reflective mirror 53 reflects the slit-shaped illumination light from the illumination optical system 10 to guide to the hyperboloidal concave mirror 54. Further, the reflective mirror 53 guides the returning light from the eye E to be examined, which is reflected by the hyperboloidal concave mirror 54, to the relay lens 40. In some embodiments, the fundus imaging apparatus 1b may have a configuration in which the reflective mirror 53 is omitted.

By deflecting the optical axis using the reflective mirror 53, the size in the depth direction (z direction) of the optical system of the fundus imaging apparatus 1b can be reduced.

(Hyperboloidal concave mirror 54)

The hyperboloidal concave mirror 54 has a concave-shaped reflective surface. The reflective surface of the hyperboloidal concave mirror 54 is a hyperboloid. The hyperboloidal concave mirror 54 is an example of the concave mirror. One of focal points of the hyperboloidal concave mirror 54 is the first focal point F1.

(Hyperboloidal convex mirror 55)

The hyperboloidal convex mirror 55 has a convex-shaped reflective surface. A reflective surface of the hyperboloidal convex mirror 55 is a hyperboloid. The hyperboloidal convex mirror 55 is an example of the convex mirror. One of focal points of the hyperboloidal convex mirror 55 is the second focal point F2.

(ellipsoidal concave mirror 56)

A reflective surface of the ellipsoidal concave mirror 56 is a concave-shaped ellipsoid. The ellipsoidal concave mirror 56 is an example of the concave mirror.

The ellipsoidal concave mirror 56 has two optically conjugate focal points (first focal point F3, second focal point F4). The ellipsoidal concave mirror 56 is arranged so that the first focal point F3 substantially coincides with the second focal point F2 of the hyperboloidal convex mirror 55. In some embodiments, the ellipsoidal concave mirror 56 is arranged so that the first focal point F3 substantially coincides with a position conjugate optically to the second focal point F2 of the hyperboloidal convex mirror 55 (conjugate position of the second focal point F2) or near the position. The eye E to be examined is arranged at the second focal point F4 of the ellipsoidal concave mirror 56. In other words, the ellipsoidal concave mirror 56 is positioned so that the second focal point F4 substantially coincides with eye to be examined position where the eye E to be examined is positioned.

In such a configuration, the slit-shaped illumination light transmitted through the relay lens 40 is focused on the first focal point F1, is reflected on the reflective surface of the reflective mirror 53, is reflected on the concave-shaped reflective surface of the hyperboloidal concave mirror 54, is reflected on the convex-shaped reflective surface of the hyperboloidal convex mirror 55, is reflected on the reflective surface of the ellipsoidal concave mirror 56, and is guided to the eye E to be examined arranged at the second focal point F4 of the ellipsoidal concave mirror 56.

The illumination light that has been guided to the eye E to be examined enters the eye through the pupil and is irradiated onto the fundus Ef. The returning light from the fundus Ef (eye E to be examined) is emitted outside of the eye E to be examined through the pupil, travels in the same path as the outward path in the opposite direction, and is guided to the first focal point F1 of the hyperboloidal concave mirror 54. The returning light that has been guided to the first focal point F1 is transmitted through the relay lens 40, passes through the hole part formed in the hole mirror 30, and is guided to the imaging optical system 20, as described above.

In some embodiments, at least one of the hyperboloidal concave mirror 54 or the hyperboloidal convex mirror 55 is a paraboloidal mirror. In some embodiments, at least one of the hyperboloidal concave mirror 54, the hyperboloidal convex mirror 55, or the ellipsoidal concave mirror 56 is formed so that the reflective surface is a free-form surface.

<Third modification example>

The configuration of the fundus imaging apparatus according to the embodiments is not limited to the configuration according to the embodiments or the modification examples thereof described above. For example, in the embodiments or the modification examples thereof, the hole mirror 30 may be disposed at the first focal point F1 of the first ellipsoidal concave mirror 51 or the hyperboloidal concave mirror 54, without providing the relay lens 40.

In the following, the fundus imaging apparatus according to a third modification example of the embodiments will be described focusing on differences from the fundus imaging apparatus 1 according to the embodiments.

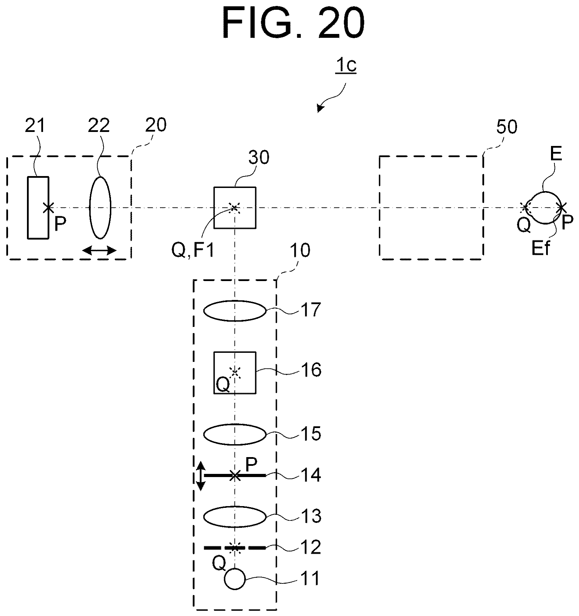

FIG. 20 shows an example of a configuration of an optical system of a fundus imaging apparatus according to the third modification example of the embodiments. In FIG. 20, like reference numerals designate like parts as in FIG. 1, and the redundant explanation may be omitted as appropriate.

The configuration of the fundus imaging apparatus 1c according to the third modified example differs from that of the fundus imaging apparatus 1 according to the embodiment in that the relay lens 40 has been removed from the configuration of the fundus imaging apparatus 1 according to the embodiment, and that the hole part formed in the hole mirror 30 is disposed at the first focal point F1 of the first ellipsoidal concave mirror 51 (not shown in FIG. 20).

The operation of the fundus imaging apparatus 1c according to the third modification example is similar to the operation of the fundus imaging apparatus 1 according to the embodiments. Thus, the detailed description of the operation will be omitted.

According to the third modification example, the same effect as the embodiment can be obtained while reducing the size of the configuration of the optical system compared to the embodiments.

<Others>

In the above embodiments or the modification examples thereof, a case where the light receiving results of the returning light have been acquired using rolling shutter system has been described. However, the configuration of the fundus imaging apparatus according to the embodiments is not limited to this.

For example, the fundus imaging apparatus may include a movable slit configured using hardware such as a focal plane shutter, which is disposed in the vicinity of the light receiving surface of the image sensor 21 or at a position substantially conjugate optically to this light receiving surface. In this case, an opening formed in the slit 14 is a rectangular shape, and a curved-shaped opening such as that shown in FIG. 10A is formed in the movable slit. In this case, the readout control of the light receiving results acquired by the image sensor 21 can be simplified.

[Actions]

The fundus imaging apparatus according to the embodiments or the modification examples thereof will be described.

The first aspect of the embodiments includes two or more curved mirrors (first ellipsoidal concave mirror 51 and second ellipsoidal concave mirror 52, or reflective mirror 53, hyperboloidal concave mirror 54, hyperboloidal convex mirror 55, and ellipsoidal concave mirror 56), an illumination optical system (10), and an image sensor (21). The illumination optical system includes a slit (14) with an opening (SL) configured to be disposed at a fundus conjugate position (P). Here, the fundus conjugate position is substantially conjugate optically to a fundus (Ef) of an eye (E) to be examined. And, the illumination optical system is configured to irradiate slit-shaped illumination light, that is generated by irradiating light from a light source onto the slit, onto the fundus through the two or more curved mirrors. The image sensor is configured to be disposed at the fundus conjugate position and to receive returning light from the eye to be examined. At least one of both ends of the opening in a longitudinal direction is displaced in a shorter direction of the opening with reference to the longitudinal direction passing through a center (OPc) of the opening.

According to such an aspect, the distortion of the image of the opening of the slit projected onto the fundus can be eliminated or reduced. Thereby, the contrast in images acquired by the image sensor can be improved.

In the second aspect of the embodiments, in the first aspect, the opening is configured to include a portion that changes in a curved shape from the center of the opening toward at least one of the both ends.

According to such an aspect, the contrast in images acquired by the image sensor can be further improved.

In the third aspect of the embodiments, in the second aspect, each of the two or more curved mirrors has one or more focal points, and the two or more curved mirrors are arranged so as to share at least one of two or more focal points.

According to such an aspect, the image formed by the illumination light can be relayed with high accuracy with a simple configuration. Thereby, higher-quality images of the fundus can be acquired.

In the fourth aspect of the embodiments, in the third aspect, the two or more curved mirrors include an ellipsoidal concave mirror (first ellipsoidal concave mirror 51 or second ellipsoidal concave mirror 52).

According to such an aspect, the image formed by the illumination light can be relayed with high accuracy using the ellipsoidal concave mirror. Thereby, higher-quality images of the fundus can be acquired.

In the fifth aspect of the embodiments, in the fourth aspect, the two or more curved mirrors are a first ellipsoidal concave mirror (51) and a second ellipsoidal concave mirror (52). The two or more curved mirrors are configured so that a first focal point (F1) of the first ellipsoidal concave mirror is configured to be arranged at an iris conjugate position substantially conjugate optically to an iris of the eye to be examined, that a second focal point (F2) of the first ellipsoidal concave mirror is configured to approximately coincide with a third focal point (first focal point F3) of the second ellipsoidal concave mirror, and that a fourth focal point (second focal point F4) of the second ellipsoidal concave mirror is arranged at the iris.

According to such an aspect, wide-angle images of the fundus can be acquired with higher image quality, using the two ellipsoidal concave mirrors.

In the sixth aspect of the embodiments, in the fifth aspect, at least one of the both ends is displaced in accordance with a maximum value of an absolute value of a difference between an incidence angle of the illumination light on the first ellipsoidal concave mirror and an incidence angle of the illumination light on the second ellipsoidal concave mirror within an irradiation range of the illumination light on the first ellipsoidal concave mirror and the second ellipsoidal concave mirror.

According to such an aspect, the opening having a shape corresponding to the incidence angle of the illumination light entering the first ellipsoidal concave mirror is formed in the slit. Thereby, the distortion of the image of the opening of the slit projected onto the fundus can be eliminated or reduced.

In the seventh aspect of the embodiments, in any one of the first aspect to the sixth aspect, the illumination optical system includes an optical scanner (16) configured to be disposed at an iris conjugate position substantially conjugate optically to an iris of the eye to be examined, and is configured to be capable of moving an irradiated position on the fundus with the illumination light by deflecting the illumination light using the optical scanner.

According to such an aspect, the high-quality image of the fundus of the eye to be examined can be acquired with a simple configuration, using the deflection control of the illumination light using the optical scanner.

In the eighth aspect of the embodiments, in the seventh aspect, the image sensor is an image sensor using a rolling shutter system.

According to such an aspect, higher-quality images of the fundus can be acquired with a simple configuration.

In the ninth aspect of the embodiments, in any one of the first aspect to the sixth aspect, the slit is configured to be movable in an optical axis direction of the illumination optical system in accordance with a dioptric power of the eye to be examined.

According to such an aspect, the fundus can be illuminated with high-intensity illumination light regardless of the dioptric power of the eye to be examined.

The tenth aspect of the embodiments, in any one of the first aspect to the sixth aspect, further includes an iris aperture (12) in that an opening is formed at a position eccentric from an optical axis, and configured to be disposed an iris conjugate position substantially conjugate optically to an iris of the eye to be examined, and an optical path dividing member (hole mirror 30) arranged between the two or more curved mirrors and the image sensor, and configured to spatially divide an optical path of the illumination light and an optical path of the returning light.

According to such an aspect, the pupil dividing between the illumination light incident within the eye and the returning light emitted from inside the eye to the outside can be performed, with a simple configuration. Thereby, higher-quality images of the fundus can be acquired.

<Others>

The embodiment described above is merely an example for implementing the present invention. Those who intend to implement the present invention can apply any modification, omission, addition, or the like within the scope of the gist of the present invention.

In some embodiments, a program for causing a processor (computer) to execute the method of controlling the fundus imaging apparatus described above is provided. Such a program can be stored in any non-transitory recording medium (storage medium) that can be read by a computer. Examples of the recording medium include a semiconductor memory, an optical disk, a magneto-optical disk (CD-ROM, DVD-RAM, DVD-ROM, MO, etc.), a magnetic storage medium (hard disk, floppy (registered trade mark) disk, ZIP, etc.), and the like. Further, the program may be transmitted and received through a network such as the Internet, LAN, etc.

[Explanation of symbols]

1, 1a, 1b, 1c Fundus imaging apparatus
10 Illumination optical system
11 Illumination light source
12 Iris aperture
14 Slit
16 Optical scanner
20 Imaging optical system
21 Image sensor

30 Hole mirror
50 Relay optical system
51 First ellipsoidal concave mirror
52 Second ellipsoidal concave mirror
60, 60a Controller
61, 61a Main controller
62, 62a Storage unit
70, 70a Image forming unit
71, 71a Image distortion correction unit
75a Data processor
80 UI unit
90 Dichroic mirror
100 OCT optical system
E Eye to be examined
Ef Fundus
F1, F3 First focal point
F2, F4 Second focal point
P Fundus conjugate position
Q Iris conjugate position

**Claims**

1. A fundus imaging apparatus, comprising:

   two or more curved mirrors;
   an illumination optical system including a slit with an opening configured to be disposed at a fundus conjugate position, the fundus conjugate position being substantially conjugate optically to a fundus of an eye to be examined, and configured to irradiate slit-shaped illumination light, that is generated by irradiating light from a light source onto the slit, onto the fundus through the two or more curved mirrors; and
   an image sensor configured to be disposed at the fundus conjugate position and to receive returning light from the eye to be examined, wherein
   at least one of both ends of the opening in a longitudinal direction is displaced in a shorter direction of the opening with reference to the longitudinal direction passing through a center of the opening.

2. The fundus imaging apparatus of claim 1, wherein
   the opening is configured to include a portion that changes in a curved shape from the center of the opening toward at least one of the both ends.

3. The fundus imaging apparatus of claim 2, wherein
   each of the two or more curved mirrors has one or more focal points, and the two or more curved mirrors are arranged so as to share at least one of two or more focal points.

4. The fundus imaging apparatus of claim 3, wherein
   the two or more curved mirrors include an ellipsoidal concave mirror.

5. The fundus imaging apparatus of claim 4, wherein

   the two or more curved mirrors are a first ellipsoidal concave mirror and a second ellipsoidal concave mirror, and
   the two or more curved mirrors are configured so that

   a first focal point of the first ellipsoidal concave mirror is arranged at an iris conjugate position substantially conjugate optically to an iris of the eye to be examined,
   a second focal point of the first ellipsoidal concave mirror approximately coincides with a third focal point of the second ellipsoidal concave mirror, and
   a fourth focal point of the second ellipsoidal concave mirror is arranged at the iris.

6. The fundus imaging apparatus of claim 5, wherein

at least one of the both ends is displaced in accordance with a maximum value of an absolute value of a difference between an incidence angle of the illumination light on the first ellipsoidal concave mirror and an incidence angle of the illumination light on the second ellipsoidal concave mirror within an irradiation range of the illumination light on the first ellipsoidal concave mirror and the second ellipsoidal concave mirror.

7.  The fundus imaging apparatus of any one of claims 1 to 6, wherein
    the illumination optical system includes an optical scanner configured to be disposed at an iris conjugate position substantially conjugate optically to an iris of the eye to be examined, and is configured to be capable of moving an irradiated position on the fundus with the illumination light by deflecting the illumination light using the optical scanner.

8.  The fundus imaging apparatus of claim 7, wherein
    the image sensor is an image sensor using a rolling shutter system.

9.  The fundus imaging apparatus of any one of claims 1 to 6, wherein
    the slit is configured to be movable in an optical axis direction of the illumination optical system in accordance with a dioptric power of the eye to be examined.

10. The fundus imaging apparatus of any one of claims 1 to 6, further comprising:

    an iris aperture in that an opening is formed at a position eccentric from an optical axis, and configured to be disposed an iris conjugate position substantially conjugate optically to an iris of the eye to be examined; and
    an optical path dividing member arranged between the two or more curved mirrors and the image sensor, and configured to spatially divide an optical path of the illumination light and an optical path of the returning light.

# FIG. 1

FIG. 2

FIG. 3

# FIG. 4

# FIG. 5

# FIG. 6

# FIG. 7

# FIG. 8

# FIG. 9A

# FIG. 9B

# FIG. 9C

# FIG. 10A

# FIG. 10B

# FIG. 10C

# FIG. 10D

# FIG. 10E

# FIG. 11A

# FIG. 11B

# FIG. 12A

Side View

Top View

# FIG. 12B

Side View

Top View

# FIG. 13

# FIG. 14

```
          ┌──────────┐
          │  START   │
          └──────────┘
               │
               ▼
    ┌─────────────────────┐
S1  │  PERFORM FOCUSING   │
    └─────────────────────┘
               │
               ▼
    ┌─────────────────────┐
S2  │  TURN ILLUMINATION  │
    │  LIGHT SOURCE ON    │
    └─────────────────────┘
               │
               ▼◄──────────────────────┐
    ┌──────────────────────────────┐   │
    │ PERFORM DEFLECTION CONTROL OF│   │
S3  │      ILLUMINATION LIGHT /    │   │
    │ SET LIGHT RECEIVABLE REGION OF│  │
    │    LIGHT RECEIVING SURFACE   │   │
    └──────────────────────────────┘   │
               │                        │
               ▼                        │
            ╱───────╲            N      │
S4       ╱ TERMINATED? ╲ ───────────────┘
         ╲           ╱
            ╲───────╱
               │ Y
               ▼
    ┌─────────────────────┐
S5  │   ACQUIRE IMAGE     │
    └─────────────────────┘
               │
               ▼
            ╱───────────╲        N
S6       ╱ PERFORM IMAGE   ╲ ───────────┐
         ╲ DISTORTION       ╱           │
            ╲ CORRECTION? ╱             │
               │ Y                      │
               ▼                        │
    ┌─────────────────────┐             │
S7  │  CORRECT IMAGE      │             │
    │  DISTORTION         │             │
    └─────────────────────┘             │
               │◄────────────────────────┘
               ▼
          ┌──────────┐
          │   END    │
          └──────────┘
```

# FIG. 15

# FIG. 16

# FIG. 17

EP 4 691 345 A1

# FIG. 18

START

S11 | SET SCAN RANGE

S12 | TURN OCT LIGHT SOURCE ON

S13 | PERFORM OCT SCAN

S14 | TERMINATED? — N

Y

S15 | FORM OCT IMAGE

END

FIG. 19

# FIG. 20

# EP 4 691 345 A1

<table>
<tr><td colspan="2" align="center">INTERNATIONAL SEARCH REPORT</td><td>International application No.<br><br>**PCT/JP2024/004745**</td></tr>
</table>

| A. | CLASSIFICATION OF SUBJECT MATTER |
|---|---|

*A61B 3/14*(2006.01)i; *A61B 3/12*(2006.01)i
FI:   A61B3/14; A61B3/12

According to International Patent Classification (IPC) or to both national classification and IPC

| B. | FIELDS SEARCHED |
|---|---|

Minimum documentation searched (classification system followed by classification symbols)

A61B3/14; A61B3/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2022/124170 A1 (KABUSHIKI KAISHA TOPCON) 16 June 2022 (2022-06-16)<br>entire text, all drawings | 1-10 |
| A | JP 2020-110224 A (KABUSHIKI KAISHA TOPCON) 27 July 2020 (2020-07-27)<br>entire text, all drawings | 1-10 |
| A | WO 2018/088338 A1 (KOWA CO., LTD.) 17 May 2018 (2018-05-17)<br>entire text, all drawings | 1-10 |
| A | JP 2018-167000 A (NIDEK KK) 01 November 2018 (2018-11-01)<br>entire text, all drawings | 1-10 |
| A | JP 59-51832 A (KABUSHIKI KAISHA TOPCON) 26 March 1984 (1984-03-26)<br>entire text, all drawings | 1-10 |
| A | JP 62-231617 A (CANON KABUSHIKI KAISHA) 12 October 1987 (1987-10-12)<br>entire text, all drawings | 1-10 |

[✓] Further documents are listed in the continuation of Box C.          [✓] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **21 March 2024** | **02 April 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**

International application No.

**PCT/JP2024/004745**

| C. | DOCUMENTS CONSIDERED TO BE RELEVANT | |
|---|---|---|
| Category* | Citation of document, with indication, where appropriate, of the relevant passages | Relevant to claim No. |
| A | JP 2009-261573 A (CANON KABUSHIKI KAISHA) 12 November 2009 (2009-11-12) entire text, all drawings | 1-10 |
| A | JP 2001-46338 A (RYUSYO INDUSTRIAL CO., LTD.) 20 February 2001 (2001-02-20) entire text, all drawings | 1-10 |

Form PCT/ISA/210 (second sheet) (July 2022)

## INTERNATIONAL SEARCH REPORT
### Information on patent family members

International application No.

**PCT/JP2024/004745**

| Patent document cited in search report | | | Publication date (day/month/year) | Patent family member(s) | | | Publication date (day/month/year) |
|---|---|---|---|---|---|---|---|
| WO | 2022/124170 | A1 | 16 June 2022 | US | 2023/0277055 | A1 | |
| | | | | entire text, all drawings | | | |
| | | | | JP | 2023-122620 | A | |
| | | | | EP | 4260794 | A1 | |
| | | | | CN | 116568202 | A | |
| JP | 2020-110224 | A | 27 July 2020 | US | 2020/0214557 | A1 | |
| | | | | entire text, all drawings | | | |
| | | | | EP | 3679855 | A1 | |
| WO | 2018/088338 | A1 | 17 May 2018 | (Family: none) | | | |
| JP | 2018-167000 | A | 01 November 2018 | US | 2018/0289260 | A1 | |
| | | | | entire text, all drawings | | | |
| JP | 59-51832 | A | 26 March 1984 | (Family: none) | | | |
| JP | 62-231617 | A | 12 October 1987 | (Family: none) | | | |
| JP | 2009-261573 | A | 12 November 2009 | US | 2009/0268160 | A1 | |
| | | | | entire text, all drawings | | | |
| | | | | CN | 101564286 | A | |
| JP | 2001-46338 | A | 20 February 2001 | (Family: none) | | | |

Form PCT/ISA/210 (patent family annex) (July 2022)

**EP 4 691 345 A1**

**Patent documents cited in the description**

- JP 2009543585 W
- US 7831106 B
- WO 2022124170 A
- JP 2017526474 W
- US 8237835 B